(19) Europäisches Patentamt
European Patent Office
Office européen des brevets

(11) Publication number: **0 314 446 B1**

(12) # EUROPEAN PATENT SPECIFICATION

(45) Date of publication of patent specification: **15.09.93**

(51) Int. Cl.5: **C07D 311/68**, C07D 405/04, C07D 407/04, A61K 31/35, C07D 493/04

(21) Application number: **88310046.3**

(22) Date of filing: **26.10.88**

The file contains technical information submitted after the application was filed and not included in this specification

(54) **Novel antihypertensive benzopyran derivatives.**

(30) Priority: **27.10.87 CA 550349**
**22.01.88 US 146875**
**24.06.88 US 210970**

(43) Date of publication of application:
**03.05.89 Bulletin 89/18**

(45) Publication of the grant of the patent:
**15.09.93 Bulletin 93/37**

(84) Designated Contracting States:
**AT BE CH DE ES FR GR IT LI LU NL SE**

(56) References cited:
**EP-A- 0 076 075**
**EP-A- 0 126 311**
**EP-A- 0 158 923**

(73) Proprietor: **AMERICAN HOME PRODUCTS CORPORATION**
**685, Third Avenue**
**New York, New York 10017(US)**

(72) Inventor: **Ouagliato, Dominick Anthony**
**1460-F Oaktree Drive**
**North Brunswick, NJ(US)**
Inventor: **Humber, Leslie George**
**126 Nathan Drive**
**North Brunswick, NJ(US)**

(74) Representative: **Wileman, David Francis Dr. et al**
**c/o Wyeth Laboratories Huntercombe Lane South**
**Taplow Maidenhead Berkshire SL6 OPH (GB)**

EP 0 314 446 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel benzopyrans having pharmacological activity, to a process for preparing them, to intermediates used in preparing them and to pharmaceutical compositions containing them. They can be used in the treatment of hypertension.

European Patent Publications 126,311 and 158,923 disclose classes of chromans that are described as having blood pressure lowering activity.

The present invention discloses compound represented by formula (I)

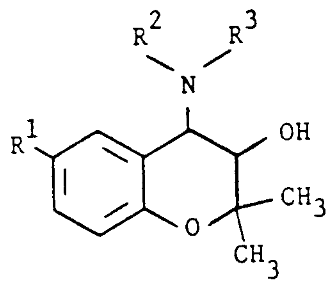

(I)

wherein $R^1$ is trifluoromethoxy or $\beta,\beta,\beta$-trifluoroethoxy; $R^2$ is selected from hydrogen, lower alkyl containing 1 to 5 carbon atoms, cyclo lower alkyl containing 5 to 8 carbon atoms,

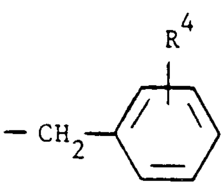 , 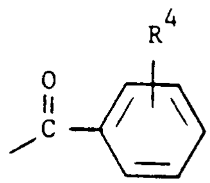 or 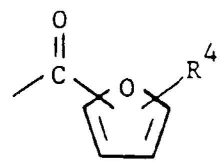 ;

$R^3$ is

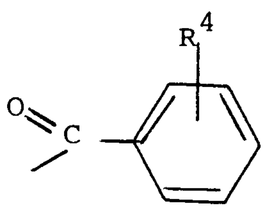 or 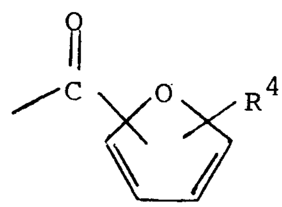

or $R^2$ and $R^3$ are joined together to form

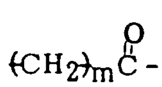

wherein m is 3 to 6; or $R^2$ and $R^3$ are joined together to form

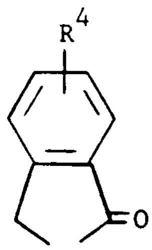

or

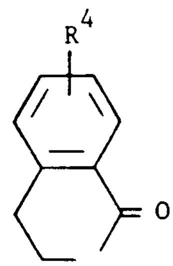

wherein $R^4$ is selected from the group consisting of hydrogen, alkoxy containing 1 to 5 carbon atoms, amino or mono- or disubstituted alkyl amino wherein said alkyl groups contain 1 to 5 carbon atoms and the pharmaceutically acceptable salts and solvates thereof.

A preferred aspect of the present invention are compounds of formula (I) wherein $R^1$ is trifluoromethoxy and $R^2$ and $R^3$ are joined to form

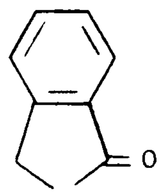

The compounds of formula (I), are asymmetric and, therefore, can exist in the form of optical isomers. The present invention extends to all such isomers individually and as mixtures, such as racemic modifications.

Preferably, a compound of formula (I) is in substantially pure form.

Examples of compounds of formula (I) include the compounds prepared in the Examples hereinafter.

An example of a solvate is hydrate, e.g. monohydrate

The present invention also provides processes for the preparation of a compound of formula (I), which comprises

(a) reacting a compound of formula

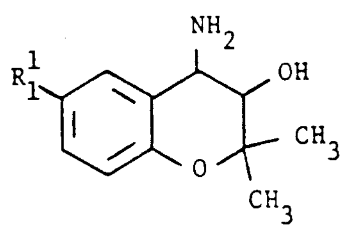

(II)

wherein $R_1{}^1$ is $R^1$ as defined hereinbefore or a group or atom convertible thereto, with a compound of formula (III)

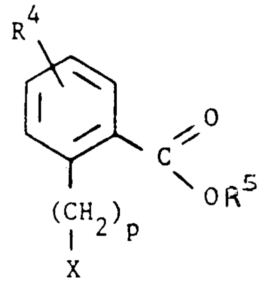

(III)

wherein X is chlorine, bromine, or iodine; $R^4$ is as defined above; p is 1 or 2 -$COOR^5$ is an ester function and if required converting the the $R_1^1$ group or atom to a group as defined by $R^1$ above to give a compound of formula I wherein $R^2$ and $R^3$ are joined together to form

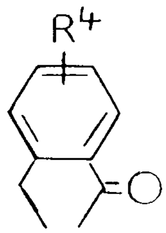

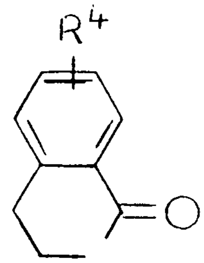

or

(b) reacting a compound of formula

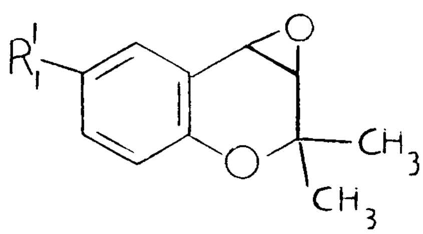

(IV)

wherein $R_1^1$ is as defined above, with a compound of formula:

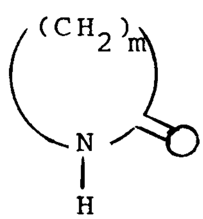

(VIII)

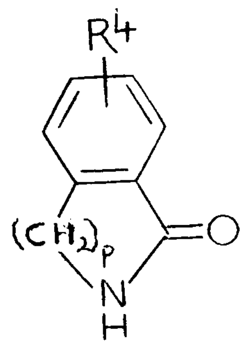 (IX)

or $NH_2-(CH_2)_m-COOR^5$ (X)

in which formulae
p is 1 or 2
m is an integer from 3 to 6,
and $COOR^5$ is an ester function,
and if required converting the $R_1^1$ , group or atom to a group as defined by $R^1$ above, to give a compound of formula I wherein $R^2$ and $R^3$ and joined to form

$-(CH_2)_mCO-$

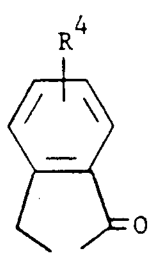 or 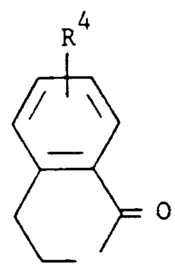

or
(c) acylating a compound of formula

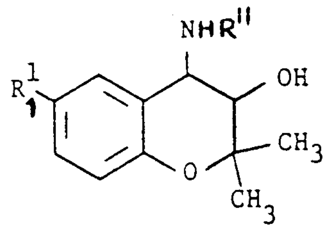 (XI)

wherein $R_1^1$ is as defined above and $R^{11}$ is hydrogen, $C_1$-$C_5$ alkyl, $C_5$-$C_8$ cycloalkyl,

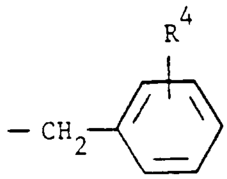 , 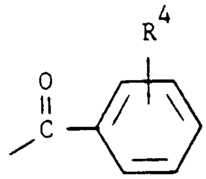 or 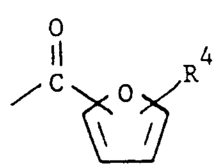 ;

with an acylating agent containing the group $R^{12}$ wherein $R^{12}$ is

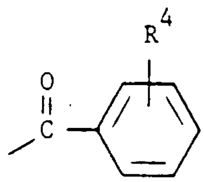

or

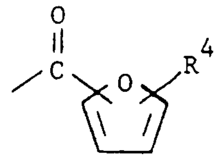

on which formulae $R^4$ is as defined above, and if required converting the $R_1^1$ group or atom to a group as defined by $R^1$ to give a compound of formula I wherein $R^2$ is $R^{11}$ as defined above and $R^3$ is $R^{12}$ as defined above;

or

(d) reacting a compound of formula II as defined above under reductive alkylation conditions with a compound of formula

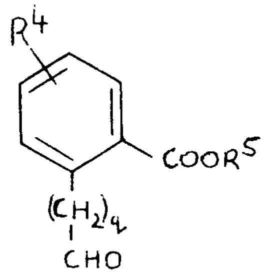

(XII)

wherein q is 0 or 1, $R^4$ and $R^5$ are as defined above and if required converting the $R_1^1$ group to $R^1$ to give a compound of formula I wherein $R^2$ and $R^3$ are joined together to form

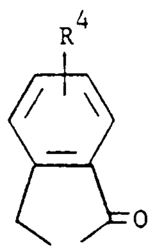

or

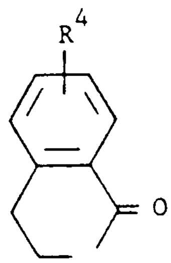

or

(e) reacting a compound of formula

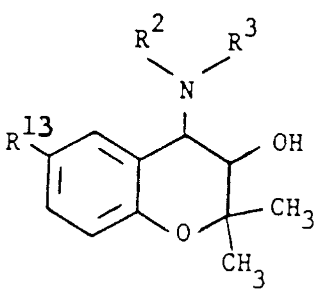

(XIII)

wherein $R^2$ and $R^3$ are as defined above and $R^{13}$ is a group or atom that can be converted directly to $R^1$, with a compound that converts $R^{13}$ to $R^{1}$,

or

(f) converting a basic compound of formula I to a pharmaceutically acceptable salt thereof by treatment with an acid or neutralising an acid addition salt with a base to give a compound of formula I in free base

form.

Starting materials which have formula I as shown above wherein $R^2$ is hydrogen, loweralkyl containing 1 to 5 carbon atoms, cyclo loweralkyl containing 5 to 8 carbon atoms or

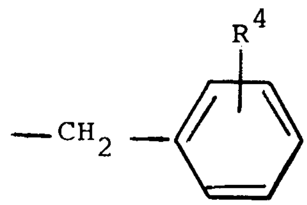

and $R^3$ is hydrogen are prepared by

(i) reducing a compound of formula

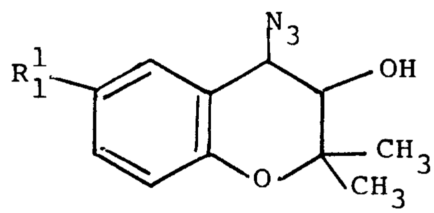

wherein $R_1^1$ is as defined above to give a compound of formula I wherein $R^2$ and $R^3$ are both hydrogen, e.g using hydrogen and a catalyst such as 10%Pd on carbon or (ii) reacting a compound of formula IV as defined above with a compound of formula $R^9NH_2$ where $R^9$ is $R^2$ as defined immediately above.

With reference to process (a) above it is particularly preferred that the reaction between the compounds of formula (II) and (III) is carried out under alkylation conditions so as to facilitate the formation of the desired bonds, for example by heating in the presence of base such as alkali metal carbonate eg. $K_2CO_3$ or a tertiary amine. Examples of $R^5$ are alkyl groups expecially those having 1 to 6 carbon atoms, eg. methyl, ethyl, propyl, butyl.

With reference to process (ii) the reaction with ammonia or an amine of formula $R^9NH_2$ is conveniently carried out, preferably with heating, in the presence of an inert solvent, eg. an alcohol such as methanol or ethanol or in the absence of a solvent where the amine is a liquid. When ammonia is used ($R^9$ is hydrogen in the above formula) then the reaction may be effected by treating the compound of formula IV with ammonium hydroxide. With reference to process (b) the reaction with an amide of formula VIII or IX is conveniently performed under strongly basic conditions for example in the presence of an alkali metal hydride or amide such as NaH or $NaNH_2$. The compound of formula X may also be reacted under strongly basic conditions. Process (b) is suitable for giving the trans isomer. With reference to process (c) above acylation is conveniently carried out under basic conditions using techniques generally known for coupling amino acids in peptide chemistry. Examples of acylating agents are reactive derivatives of acids of formula:

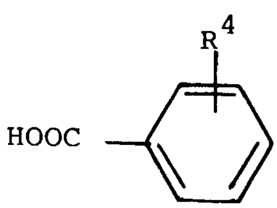

(XIV)

or

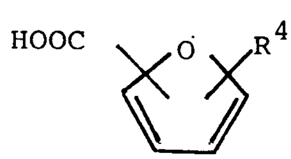

(XV)

such as acid halides, eg. the chloride, azides, anhydrides (eg. formed with carbonyldiimidazole) or activated esters (eg. 1-benzotriazolyl, 2,3,4-trichlorophenyl or p-nitrophenyl) or O acyl ureas obtained from carbodiimides such as dialkylcarbodiimides eg. dicyclohexylcarbodiimide.

Descriptions of methods for activating carboxy groups are given in general textbooks on peptide chemistry, eg. The Practise of Peptide Synthesis, by M Bodanszky and A Bodanszky Springer-Verlag 1984

Volume 21 of the series "Reactivity and Structure Concepts in Organic Chemistry".

Diacylation where the starting material of formula XII already has an acyl $R^{11}$ group or where an amino compound is to be diacylated can be carried out by using an acyl halide as acylating agent in the presence of an acid acceptor such as a molecular sieve.

With reference to process (d) above the reductive alkylation may be carried out by methods known for use with aromatic aldehydes.
See for example "The Chemistry of carbon-nitrogen double bonds" edited by Saul Patai, Interscience Publishers 1970, Chapter 6. The reaction is conveniently performed using sodium cyanoborohydride.

Examples of conversions of a group or atom from $R^1_1$ $R^{13}$ into $R^1$ are generally known in the art of synthetic chemistry. For example, if it is desired to obtain a compound of formula (I) wherein $R^1$ is a trifluoroethoxy group it is possible to convert a compound of formula (I) wherein $R^1_1$ is a hydroxy group or a protected hydroxy group to the desired trifluoroethoxy group by deprotecting the hydroxy group and alkylating the hydroxy group in a conventional manner. Examples of protecting agents and their addition and removal are generally known in the art. When $R^1$ is trifluoromethoxy then this may be formed by treating the corresponding compound where in $R^1_1$ or $R^{13}$ is $CCl_3$ O-with HF or a metal fluoride under fluorinating conditions, eg. liquid HF under pressure.

Trans compounds of formula II can be prepared by reacting an epoxide of formula IV with ammonia. Similarly trans compounds of formula XI wherein $R^{11}$ is $C_1$-$C_5$ alkyl or $C_5$-$C_8$ cycloalkyl or

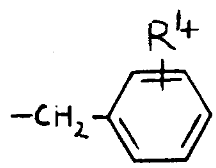

can be prepared by reacting an epoxide of formula IV with an amine of formula $R^{11}NH_2$ where $R^{11}$ is as defined immediately above.

Cis compounds of formula II can be prepared by analogy with the teaching in J. Med. Chem. 1986, Vol 29, pps 2194-2201 reacting an epoxide of formula IV with HBr to give a trans 4-bromo-3-ol compound of formula

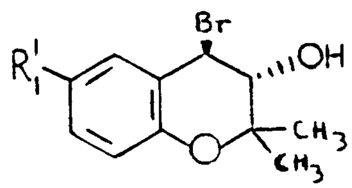

,

protecting the OH function in the form of the tetrahydropyranyl ether and reacting the protected compound with sodium azide, hydrogenating the cis azide to give the cis amine and deprotecting. Cis compounds of formula XI may be prepared by alkylating or acylating the corresponding amine of formula II by known methods.

Compounds of formula IV may be prepared by oxidising a compound of formula IVa

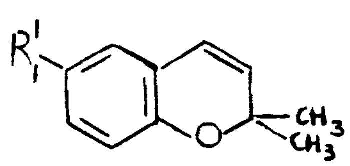

(IVa)

eg. using a peracid such as m-chloroperoxybenzoic acid. Synthetic Processes A, B and C shown herein illustrate the preparation of intermediates and final products of this invention.

In any of the processes described herein reactive substituent groups may be protected where appropriate prior to carrying out a reaction, followed by releasing the protecting group at a later or final stage. For example the 3-OH function can be protected using phthalic anhydride. The compounds of this invention are capable of forming acid addition salts with therapeutically acceptable acids. The acid addition

salts are prepared by reacting the base form of the appropriate compound of formula (I) with one or more equivalents, preferably with an excess, of the appropriate acid in an organic solvent, for example, diethyl ether or an ethanol diethyl ether mixture.

These salts, when administered to a mammal, possess the same or improved pharmacologic activities as the corresponding bases. For many purposes it is preferable to administer the salts rather than the basic compounds. Suitable acids to form these salts include the common mineral acids, eg. hydrohalic, sulfuric or phosphoric acid; the organic acids, eg. ascorbic, citric, lactic, aspartic or tartaric; and acids which are sparingly soluble in body fluids and which impart slow-release properties to their respective salts, eg pamoic or tannic acid or carboxymethyl cellulose. The preferred salt is the hydrochloride salt. The addition salts thus obtained are the functional equvalent of the parent base compund in respect to their therapeutic use. Hence, these addition salts ae included within the scope of this invention and are limited only by the requirement that the acids employed in forming the salts be therapeutically acceptable.

The compounds of formula (II) and (IV) are novel compounds included within the scope of this invention and can be prepared in accordance with the processes described herein.

The compounds of formula (III) are known compounds or can be prepared by conventional procedures from known compounds.

As mentioned previously, the compounds of formula (I) have been found useful in the treatment of hypertension.

The present invention accordingly provides a pharmaceutical composition which comprises a compound of this invention and a pharmaceutically acceptable carrier. In particular, the present invention provides an anti-hypertensive pharmaceutical composition which comprises an antihypertensive effective amount of a compound of this invention and a pharmaceutically acceptable carrier.

The compositions are preferably adapted for oral administration. However, they may be adapted for other modes of administration, for example parenteral administration for patients suffering from heart failure.

In order to obtain consistency of administration it is preferred that a composition of the invention is in the form of a unit dose. Suitable unit dose forms include tablets, capsules and powders in sachets or vials. Such unit dose forms may contain from 0.1 to 100 mg of a compound of the invention and preferably from 2 to 50 mg. Still further preferred unit dosage forms contain 5 to 25 mg of a compound of the present invention. The compounds of the present invention can be administered orally at a dose range of about 0.01 to 100 mg/kg or preferably at a dose range of 0.1 to 10 mg/kg. Such compositions may be administered from 1 to 6 times a day, more usually from 1 to 4 times a day.

The compositions of the invention may be formulated with conventional excipients, such as a filler, a disintegrating agent, a binder, a lubricant, a flavouring agent and the like. They are formulated in conventional manner, for example in a manner similar to that used for known antihypertensive agents, diuretics and $\beta$-blocking agents.

The present invention further provides a compound of the invention for use as an active therapeutic substance. Compounds of formula (I) are of particular use in the treatment of hypertension.

Synthetic Process A relates to the preparation of a compound of formula (I)

## Synthetic Process A

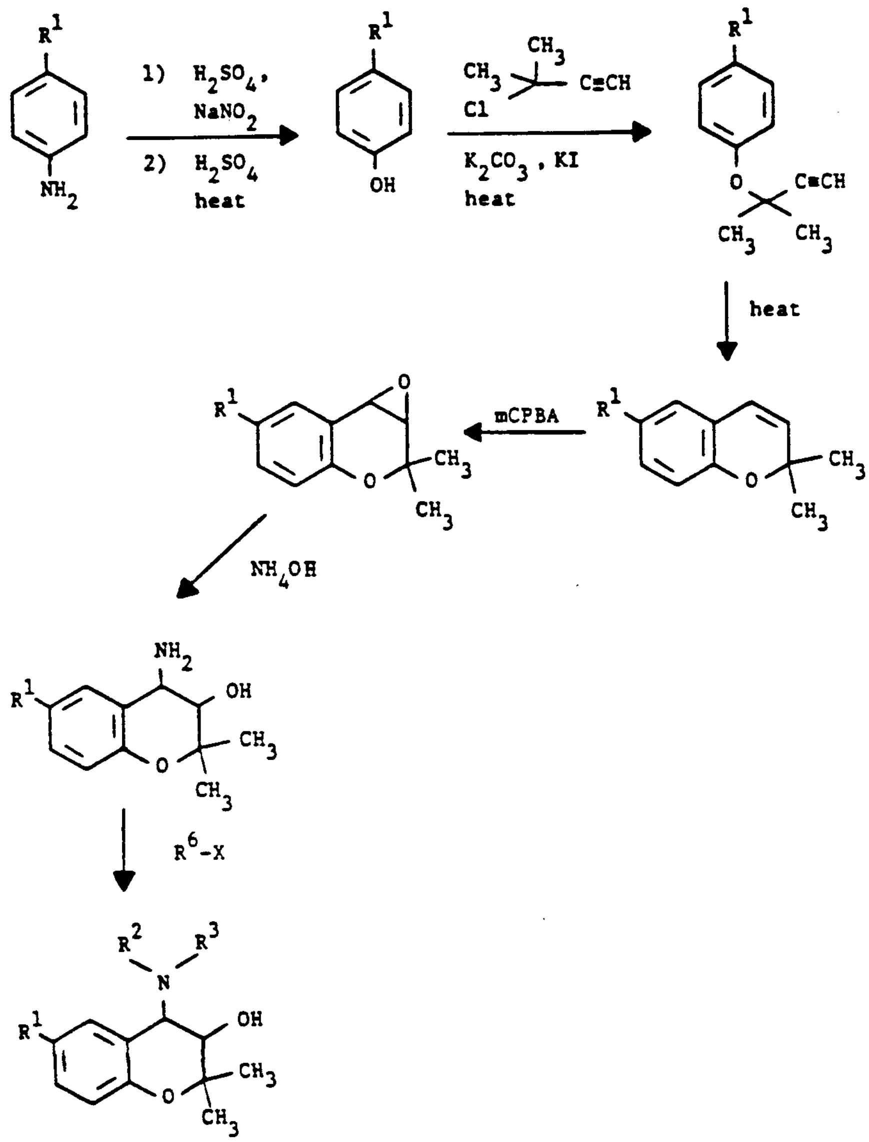


wherein when $R^2$ is hydrogen $R^3$ is benzoyl, furoyl or $R^2$ and $R^3$ are joined to form isoquinolone or isoindolone

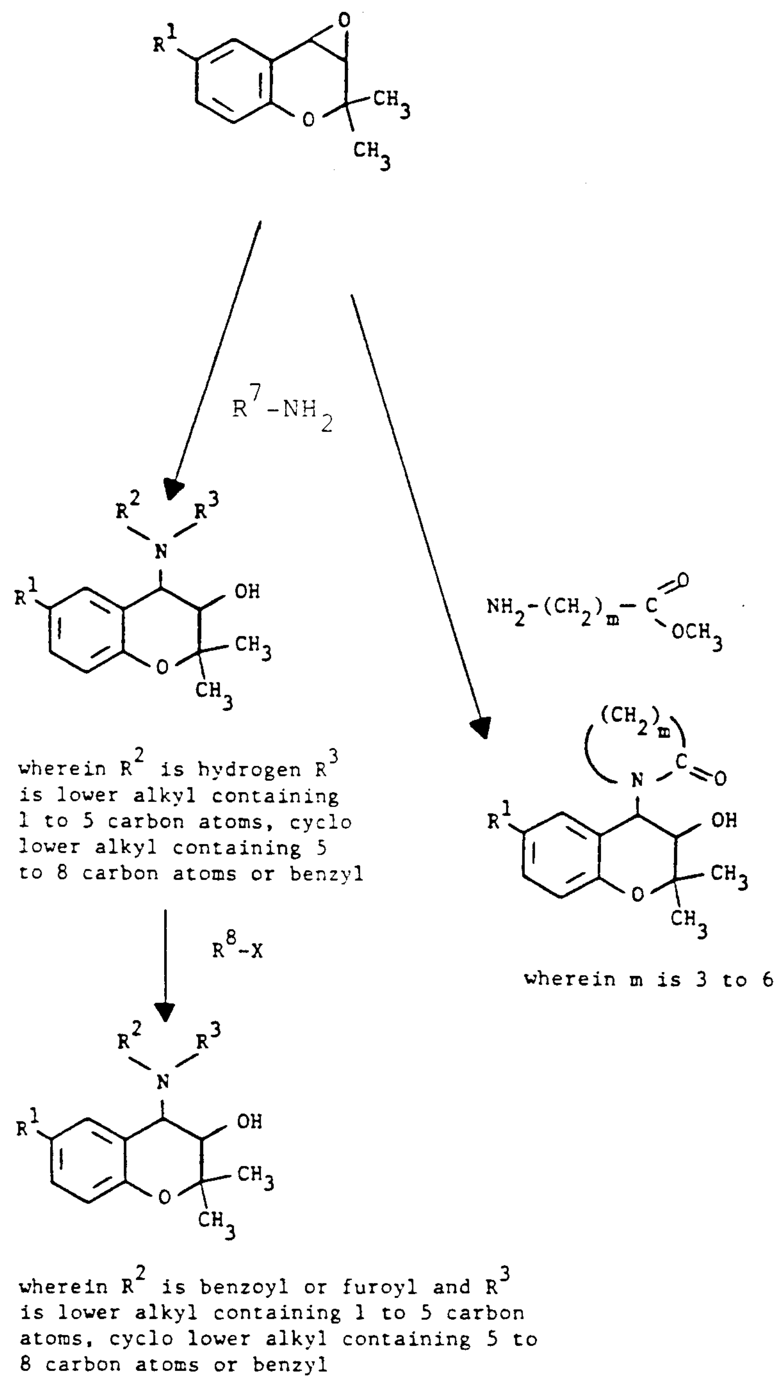


wherein $R^1$ is as defined above; X is chlorine, bromine or iodine; $R^6$ is benzoyl, furoyl, or

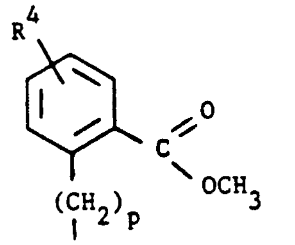

wherein p is 1 or 2; $R^7$ is lower alkyl containing 1 to 5 carbon atoms, cyclo lower alkyl containing 5 to 8 carbon atoms, or benzyl; $R^8$ is benzoyl or furoyl; and $R^4$ is as defined above.

The production of preferred compounds of the present invention is illustrated by Synthetic Process B.

## Synthetic Process B

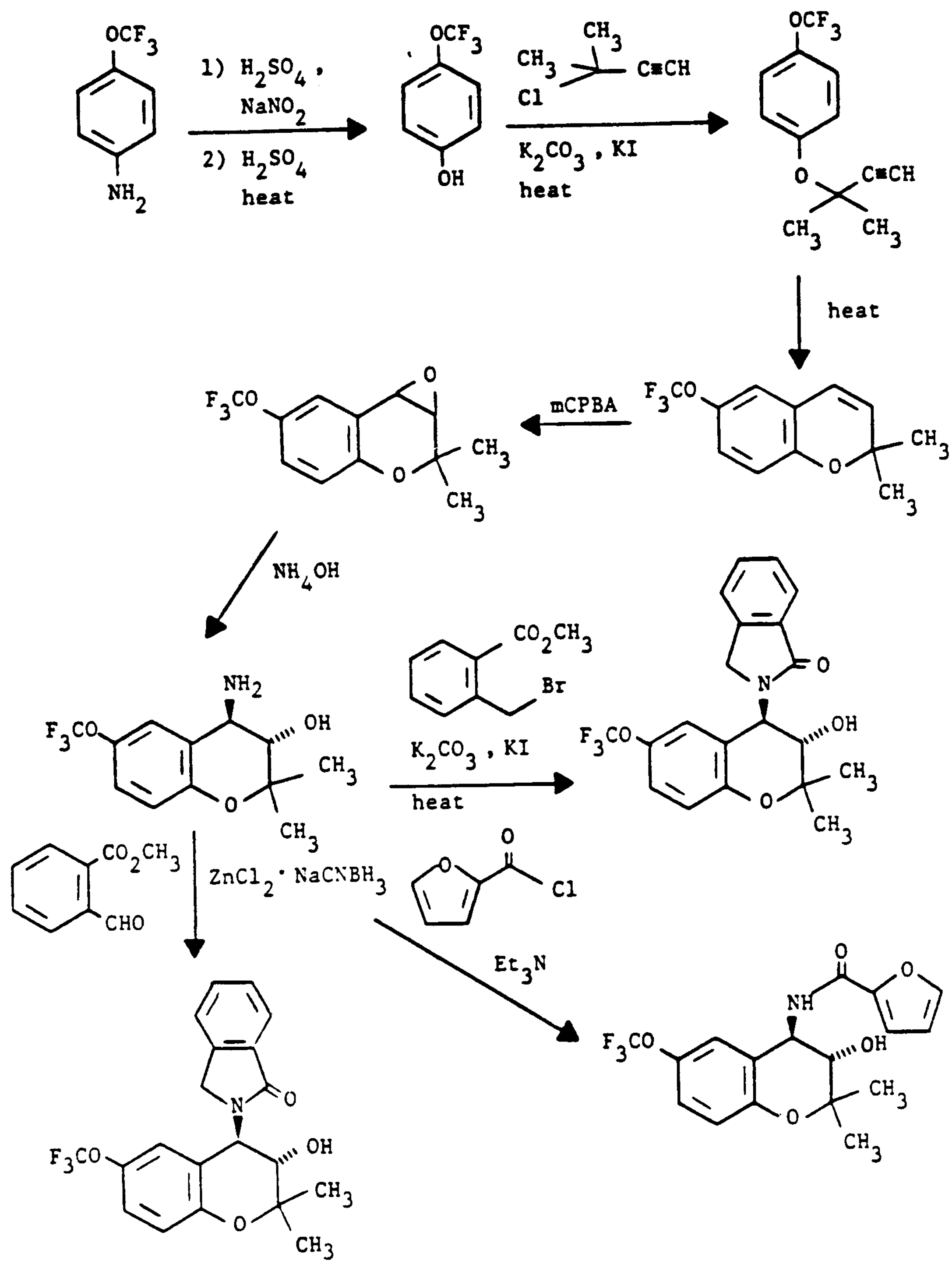


The resolution of compounds of formula (I) into optical isomers may be accomplished by reacting the racemate with an optically pure chiral auxiliary, preferably l-(l-naphthyl)ethyl isocyanate or α-methylbenzyl isocyanate, to form a mixture of two diastereomers. These diastereomers are then separated by physical

means, such as chromatography or crystallization. Each is reacted to remove the chiral auxiliary to afford the enantiomers of compounds of formula (I).

Synthetic Process C relates to the resolution of a preferred compound of formula (I).

## Synthetic Process C

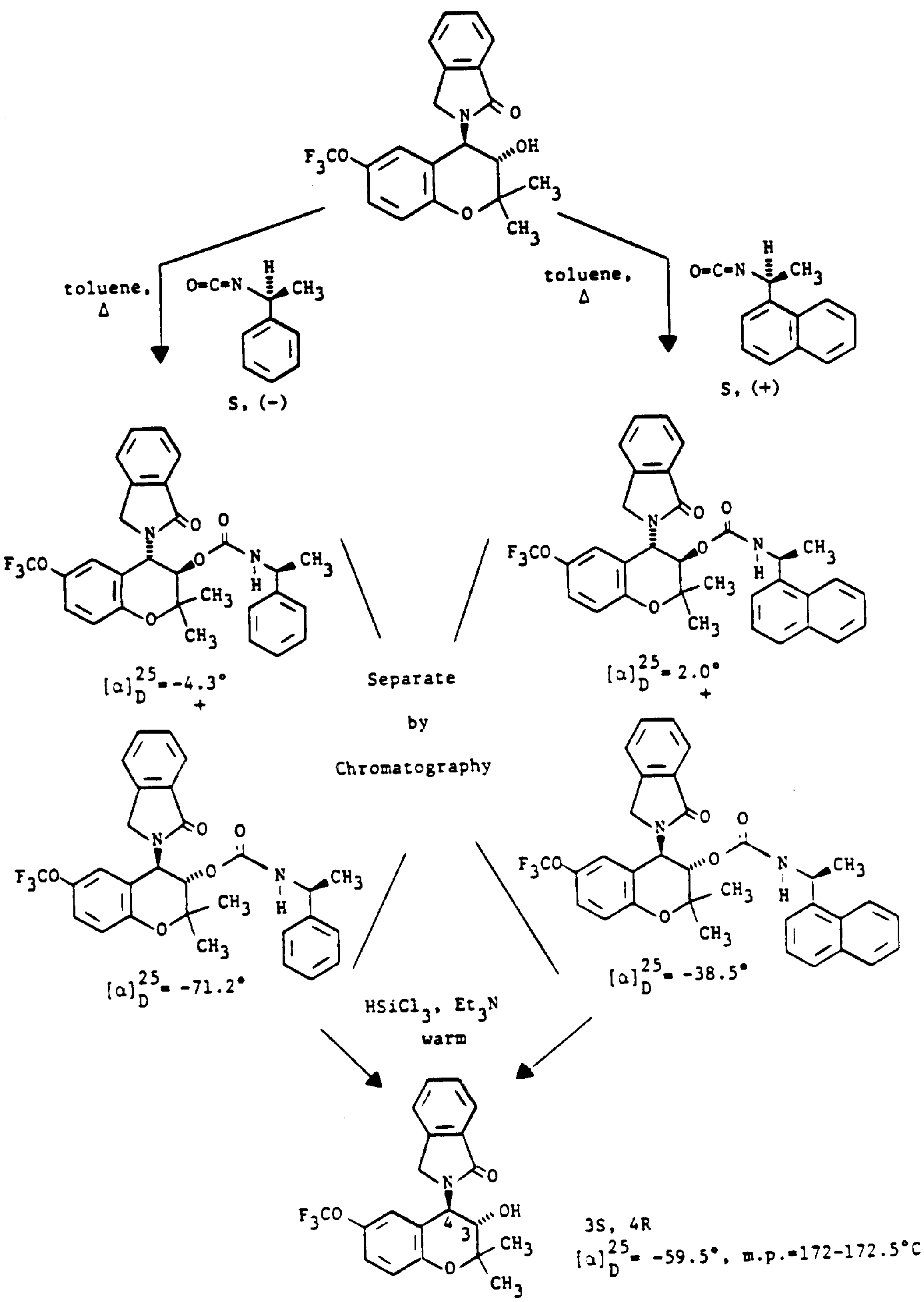


The following Examples further illustrate this invention.

EXAMPLE 1

Preparation of p-Trifluoromethoxy Phenol

p-Trifluoromethoxy aniline (49.60 g) was added rapidly dropwise to vigorously stirred 9N aqueous $H_2SO_4$ (500 mL) at 40° C. The mixture was heated to dissolve the solid, then cooled to 0° C. To the fine white suspension, a solution of sodium nitrite (19.46 g in 50 mL of $H_2O$) was added portionwise until an immediate positive KI/starch test result was obtained. This cold solution of diazonium salt was added rapidly dropwise to 9N aqueous $H_2SO_4$ (500 mL) at 110° C. Stirring and heating was continued for 2.5 hours. The mixture was cooled to 10° C and extracted with diethyl ether (3 x 500 mL). The combined organic layers were dried ($MgSO_4$), filtered and evaporated in vacuo, then flash chromatographed on $SiO_2$ using diethyl ether as eluant to give 35.0 g of the desired phenol as a light brown oil. The oil was distilled (b.p. = 75-80°C at 20 torr.) to afford a yellow liquid.
NMR ($CDCl_3$): δ 5.06 (1H, s), 6.83 (2H, d, J = 9.2), 7.11 (2H, d, J = 9.2Hz)

EXAMPLE 2

Preparation of 1-[(1,1-Dimethyl-2-propynyl)oxy)-4-(trifluoromethoxy)benzene

To a solution of p-trifluoromethoxy phenol (30.69 g), and 2-methyl-2-chloro-3-butyne (53.00 g) in dry acetonitrile (350 mL) was added potassium iodide (14.30 g) followed by potassium carbonate (95.25 g). This reaction mixture was heated at 70-80°C for four days then cooled to room temperature and filtered through celite. The precipitate was washed with dichloromethane and the washings were added to the acetonitrile. The organics were evaporated in vacuo and the oil was taken up in 250 mL of dichloromethane. The organics were washed with water (2 x 100 mL) and dilute aqueous sodium thiosulfate (2 x 100 mL), dried ($MgSO_4$), filtered and evaporated in vacuo to leave a dark brown-orange oil. Flash column chromatography on $SiO_2$ using hexane/$Et_2O$ (5/l) afforded 34.73 g of the pure product.
NMR ($CDCl_3$) δ 1.64 (6H, s), 2.60 (1H, s), 7.05-7.30 (4H, m)

EXAMPLE 3

Preparation of 2,2-Dimethyl-6-(trifluoromethoxy)-2H-1-benzopyran

A solution of 1-[(1,1-dimethyl-2-propynyl)oxy]-4-trifluoromethoxybenzene (16.25 g) in 60 mL of quinoline was heated to 175°C for 2 hours. The solution was cooled to room temperature then ether (250 mL) was added. This mixture was stirred for 15 minutes then decanted from any precipitated tars. The ether solution was washed with 1N aqueous hydrochloric acid (3 x 200 mL) then water (1 x 200 mL) and dried ($K_2CO_3$). The filtered ether solution was evaporated and flash chromatographed on $SiO_2$ using hexane/ethyl acetate (5/1) as eluant to afford 13.92 g (85%) of the desired bicyclic compound.

Alternate Preparation of 2,2-Dimethyl-6-(trifluoromethoxy)-2H-1-benzopyran

A solution of the 1-[(1,1-dimethyl-2-propynyl)oxy]-4-trifluoromethoxybenzene (29.05 g) in 100 mL of chlorobenzene (b.p. = 132°C) was heated to reflux for 24 hours. The reaction mixture was cooled and the solvent removed in vacuo. The oily residue was flash chromatographed on $SiO_2$ using hexane/ethyl acetate (5/l) as eluant to afford 19.72 g of the desired bicyclic compound.
NMR ($CDCl_3$) δ 1.42 (6H, s), 5.67 (1H, d, J = 10Hz), 6.28 (1H, d, J = 10Hz), 6.78 (1H, d, J = 5.5Hz), 6.83 (1H, d, J = 2H), 6.94 (1H, dd, J = 5.5Hz, 2Hz)

EXAMPLE 4

Preparation of 1a, 7b-Dihydro-2,2-dimethyl-6-(trifluoromethoxy)-2H-oxireno[c][l]benzopyran

To a solution of 2,2-dimethyl-6-trifluoromethoxy-2H-1-benzopyran (14.37 g) in dichloromethane (40 mL) at 0° C was added a solution of m-chloroperoxybenzoic acid (mCPBA) (14.22 g) in dichloromethane (160 mL) dropwise. After the addition was complete the ice bath was removed and the temperature allowed to warm slowly to 15° C whilst stirring for 18 hours. The reaction mixture was filtered, and the precipitate was washed with dichloromethane (50 mL). The combined filtrate was washed with 25% aqueous sodium

thiosulfate (2 x 100 mL), and 50% aqueous sodium bicarbonate (2 x 100 mL), dried ($MgSO_4$),filtered and evaporated in vacuo. The orange oil was flash chromatographed on $SiO_2$ using hexane/ether (4/1) as eluant to afford 13.36 g of the epoxide as a light yellow oil, which solidified upon standing.
NMR ($CDCl_3$) δ 1.25 (3H, s), 1.58 (3H, s), 3.49 (1H, d, J = 4Hz), 3.86 (1H, d, J = 4Hz), 6.78 (1H, d, J = 8.5Hz), 7.11 (1H, dd, J = 8.5Hz and 2Hz), 7.22 (1H, d, J = 2Hz)

## EXAMPLE 5

Preparation of trans-2,3-Dihydro-2,2-dimethyl-3-hydroxy-6-(trifluoromethoxy)-2H-1-benzopyran-4-amine

To a solution of 1a,7b-dihydro-2,2-dimethyl-6-(trifluoromethoxy)-2H-oxireno[c][l]benzopyran (6.18 g) in absolute ethanol (30 mL) at 0 °C was added ammonium hydroxide (45 mL). The reaction mixture was capped with a rubber septum and stirred for four days. The reaction mixture was evaporated in vacuo to remove ethanol and water and the oil was taken up in dichloromethane, dried ($Na_2SO_4$) filtered and concentrated in vacuo. The residue was flash chromatographed on $SiO_2$ using dichloromethane/methanol (5/1) as eluant to afford the amino-alcohol, m.p. 176-182 ° C (dec.) recrystallized from chloroform.

Two of the above reactions were run simultaneously to obtain 8.95 g of product.
NMR (DMSO-$d_6$) δ 1.07 (3H, s), 1.35 (3H, s), 3.20 (1H, d, J = 9.2Hz), 3.52 (1H, d, J = 9.2Hz), 6.76 (1H, d, J = 9Hz), 7.08 (1H, dd, J = 9Hz, 1.5Hz), 7.51 (1H, d, J = 1.5Hz)

## EXAMPLE 6

Preparation of trans-2-[2,3-Dihydro-2,2-dimethyl-3-hydroxy-6-(trifluoromethoxy)-4H-1-benzopyran-4-yl]-2,3-dihydro-1H-isoindol-1-one

To a solution of trans-2,3-dihydro-2,2-dimethyl-3-hydroxy-6-(trifluoromethoxy)-2H-1-benzopyran-4-amine (13.86 g) and methyl 2-formylbenzoate (9.03 g) in 200 mL of dry methanol was added 120 mL of a 0.5 molar solution of zinc chloride-sodium cyanoborohydride (0.06 moles eash) in dry methanol. After one hour the mixture was warmed to 50-55 ° C and held there with stirring for 14 hours.

The cooled reaction mixture was quenched with 120 mL of saturated aqueous sodium bicarbonate and the methanol was removed in vacuo. 120 mL of water was added to the residue which was then extracted with dichloromethane (3 x 200 mL). The combined extracts were washed with water (2 x 300 mL), dried over $K_2CO_3$, filtered then evaporated to leave an off-white solid.

This solid was dissolved in 500 mL of hot toluene; the mixture was then heated to reflux for 4 to 5 hours. The solution was then cooled and a white precipitate began to form. The mixture was cooled to 0 °C for 0.5 hours during which time a thick mass of white crystals formed. These crystals were collected by vacuum filtration, washed with hexane/toluene (4/1) and dried in vacuo to yield 18.30 g (93%) of analytically pure product as a white flocculent solid, m.p. 212-213 °C.

Alternate Preparation of trans-2-[2,3-Dihydro-2,2-dimethyl-3-hydroxy-6-(trifluoromethoxy)-4H-1-benzopyran-4-yl] -2,3-dihydro-1H-isoindol-1-one

To a solution of trans-2,3-dihydro-2,2-dimethyl-3-hydroxy-6-(trifluoromethoxy)-2H-1-benzopyran-4-amine (3.85 g) and methyl 2-bromomethylbenzoate (3.11 g) in dry acetonitrile (80 mL) was added potassium iodide (1.13 g) then potassium carbonate (powdered, 5.63 g). The reaction mixture was stirred under nitrogen at room temperature for 1 hour then heated in a 75-80 ° C oil bath for 24 hours. The cooled mixture was vacuum filtered through celite. The precipitate was washed with ethyl acetate (75 mL), and the filtrates were combined and evaporated. The residue was taken up in ethyl acetate (175 mL), washed with water (2 x 100 mL) then 25% aqueous sodium thiosulfate (2 x 100 mL), dried ($MgSO_4$), filtered and evaporated in vacuo. The resultant oil was crystallized from dichloromethane/ethyl acetate (5/1). The crystals were collected, washed with ether, and dried in vacuo to afford the desired compound in 48% yield, m.p. 212-213 °C.
NMR (DMSO-$d_6$) δ 1.24 (3H, s), 1.46 (3H, s), 3.91 (1H, br), 4.06 (1H, br d), 4.48 (1H, br d), 5.24 (1H, br s), 5.77 (1H, d, J = 5.8Hz), 6.70 (1H, br s), 6.92 (1H, d, J = 8.9Hz), 7.17 (1H, dd, J = 8.9Hz and 2.6Hz), 7.50-7.66 (3H, m), 7.78 (1H, d, J = 7.5Hz)

| | | | |
|---|---|---|---|
| Anal. Calcd.: | C, 61.07; | H, 4.61; | N, 3.56 |
| Found: | C, 60.92; | H, 4.87; | N, 3.35 |

EXAMPLE 7

Preparation of trans-N-[2,3-Dihydro-2,2-dimethyl-3-hydroxy-6-(trifluoromethoxy)-4H-1-benzopyran-4-yl]-2-furancarboxamide

To a solution of trans-2,3-dihydro-2,2-dimethyl-3-hydroxy-6-(trifluoromethoxy)-2H-1-benzopyran-4-amine (3.40 g) and triethylamine (1.84 mL) in dichloromethane (60 mL) at 5° C was added 2-furoyl chloride (1.30 mL) dropwise via a pipet. After 10 minutes the ice water bath was removed and the reaction was stirred and allowed to raise to ambient temperature. TLC at 2.5 hours indicated complete reaction and the mixture was increased in volume by adding 60 mL of dichloromethane. The organics were washed with 0.1N aqueous HCl (2 x 80 mL), 50% aqueous sodium bicarbonate (2 x 80 mL) and water (1 x 80 mL), dried ($MgSO_4$), filtered and evaporated in vacuo. The oily residue was flash chromatographed on $SiO_2$ using dichloromethane/ethyl acetate (8/1) as eluant to leave a colorless oil. The desired compound was crystallized from ether/hexane (1/1) to give white needles, m.p. 146-147°C yield 50%.
NMR (DMSO-$d_6$) δ 1.16 (3H, s), 1.39 (3H, s), 3.74 (1H, dd, J=5.9Hz and 9.8Hz), 4.95 (1H, t, J=9.3Hz), 5.65 (1H, d, J=6Hz), 6.64 (1H, m), 6.86 (1H, d, J=9Hz), 6.93 (1H, d, J=2.6Hz), 7.14 (1H, dd, J=9Hz and 2.6Hz), 7.17 (1H, d, J=2.6Hz), 7.86 (1H, s), 8.73 (1H, d, J=9Hz)

| | | | |
|---|---|---|---|
| Anal. Calcd.: | C, 54.99; | H, 4.34; | N, 3.77 |
| Found: | C, 54.79; | H, 4.67; | N, 3.71 |

The following Examples illustrate the resolution of the compounds of this invention into optical isomers.

EXAMPLE 8

Preparation of (+)- and (-)-(trans)-[1-(1-naphthalenyl)ethyl]carbamic Acid 4-(1,3-Dihydro-1-oxo-2H-isoindol-2-yl)-3,4-dihydro-2,2-dimethyl-6-(trifluoromethoxy)-2H-1-benzopyran-3-yl Ester

A solution of trans-2-[2,3-dihydro-2,2-dimethyl-3-hydroxy-6-(trifluoromethoxy)-4H-1-benzopyran-4-yl]-2,3-dihydro-1H-isoindol-1-one(8.53 g) and S-(+)-1-(1-naphthyl)ethyl isocyanate (5.15 g) in dry toluene (275 mL) was heated at 110°-115° C for 24 hours. The cooled mixture was evaporated in vacuo, then flash chromatographed on silica gel (1 kg) using dichloromethane/hexane/ethyl acetate (6/6/1) as eluant to afford the following: a) 4.74 g of diastereomer A, b) 3.75 g of a mixture of both diastereomers and c) 4.20 g of diastereomer B.
Pure diastereomer A): glass, $[\alpha]_D^{25}$ = +2.0, c=1, $CHCl_3$
NMR ($CDCl_3$): δ 1.25 (3H, d), 1.33 (3H, s), 1.42 (3H, s), 4.09 (1H, AB d, J=16.4Hz), 4.59 (1H, AB d, J=16.4Hz), 5.12 (NH, d, J=8.2Hz), 5.20 (1H d, J=10.5Hz), 5.33 (1H, m), 5.76 (1H, d, J=10.5Hz), 6.78 (1H, d, J=3.0Hz), 6.88 (1H, d, J=8.9Hz), 7.07 (1H, dd, J=8.9Hz and 3.0Hz), 7.38-7.60 (7H, m) and 7.70-8.00 (4H, m)
Pure diastereomer B): glass, $[\alpha]_D^{25}$ = -38.0°, c=1, $CHCl_3$
NMR ($CDCl_3$): δ 1.40 (3H, s), 1.51 (3H, s), 1.53 (3H, d, J=7.0Hz), 3.96 (1H, AB d, J=16.3Hz), 4.40 (1H, AB d, J=16.3Hz), 5.23 (1H, d, J=10.6Hz), 5.25 (NH, d), 5.39 (1H, m), 5.76 (1H, d, J=10.6Hz) and 6.68-7.87 (14H, series of m)

In addition, this experiment was repeated using S-(-)-α-methylbenzyl isocyanate as the chiral auxiliary. The purified and separated diastereomers were crystalilzed by diffusion of hexane into an ethyl acetate solution.

EXAMPLE 9

Preparation of (-)-3S,4R-trans-2-[2,3-Dihydro-2,2-dimethyl-3-hydroxy-6-(trifluoromethoxy)-4H-1-benzopyran-4-yl]-2,3-dihydro-1H-isoindol-1-one

To a solution of (-)-(trans)-[1-(1-naphthalenyl)ethyl]carbamic acid 4-(1,3-dihydro-1-oxo-2H-isoindol-2-yl)-3,4-dihydro-2,2-dimethyl-6-(trifluoromethoxy)-2H-1-benzopyran-3-yl ester, diastereomer B, (5.86 g) in dichloromethane (150 mL) at room temperature was added triethylamine (3.49 mL) followed by the dropwise addition of trichlorosilane (2.53 mL). This mixture was stirred at room temperature for 6 hours then warmed to 40° C for 18 hours. The cooled mixture was quenched with 140 mL of 2M aqueous ammonium hydroxide. This mixture was stirred for 30 mintues. Celite was added to the mixture and then it was filtered. The precipitate was placed into a flask and washed with good agitation with dichloromethane (100 mL). This mixture was filtered and the filtrates were combined in a separatory funnel. The organic layer was washed with water, dried ($K_2CO_3$) and evaporated in vacuo. The residue was flash chromatographed on silica gel using ethyl ether/hexane (4/1) as eluant. The desired product was crystallized from hot hexane/ethyl ether (2/1), m.p. 172-172.5°C.

$[\alpha]_D^{25}$ = -59.50°, c=1, $CHCl_3$

The NMR of this product is substantively the same as for the product of Example 6.

Pharmacological Data

Male Okamoto-Aoki spontaneously hypertensive rats (SHR) ranging in weight from 250-400 g were anesthetized with halothane. Their left femoral arteries and veins were cannulated with polyethylene tubing of the appropriate size (id. 0.023", o.d. 0.038"). Each animal was placed in a Bollman cage, and the tail, along with two cannulas, was extended through a hole in one end of the cage. The tail was taped securely to a firm rubber board to prevent the rat from turning in its cage to dislodge the cannulas. The femoral arterial cannula was connected to a Statham pressure transducer which in turn was attached to a polygraph for recording arterial pressure and pulse rate. The pulse rate was considered to be the heart rate.

After the blood pressure has stabilized (usually 2 hours after cessation of the anesthesia), standard agonists were injected by the i.v. route. The doses administered were: isoproterenol 0.5 µg/kg, adrenaline 2.0 µg/kg, tyramine 200 µg/kg and angiotensin-I 0.25 µg/kg. The agonists were given in random order except that tyramine was never preceded by isoproterenol as the response to tyramine seemed to be blunted after a prior injection of isoproterenol. Enough time was allowed for the BP to return to preinjection levels before the test compound was administered by gastric lavage. The time of drug administration was designated as time zero. Heart rate and blood pressure were recorded at 5, 10, 15, 30, 45 and 60 minutes and hourly thereafter for a period of 4 hours after drug administration. At 1 and 2 hours post-drug the agonists were again injected at the same concentration and in the same order as during the control period.

For each compound the maximum mean fall in blood pressure was compared to pretreatment control values and expressed as a percentage fall in blood pressure.

## Blood Pressure Lowering by Compound of Formula (I)

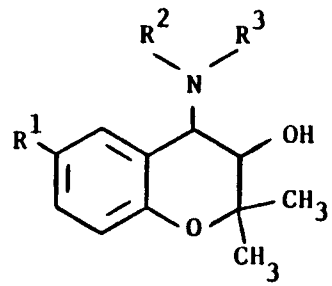

(I)

| $R^1$ | $R^2$ | mg/kg p.o. | n | Blood Pressure: Pre-treat. MABP mm Hg | Blood Pressure: Max Δ mm Hg | Blood Pressure: Max Δ BP % | Heart Rate: Pre-treat. HR beats/min. | Heart Rate: Max Δ beats | Heart Rate: Max Δ HR % |
|---|---|---|---|---|---|---|---|---|---|
| $CF_3O-$ | 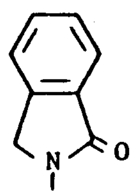 racemate | 10 | 3 | 178 | -93 at 4Hr | -52 | 351 | +87 at 4Hr | +25 |
| | | 0.5 | 4 | 181 | -56 at 4Hr | -31 | 364 | +89 at 4Hr | +24 |
| | 3S, 4R enantiomer | 0.05 | 6 | 183 | -13 at 3Hr | -7 | 415 | -11 at 3Hr | -3 |
| | | 0.13 | 8 | 178 | -35 at 2Hr | -20 | 369 | +34 at 2Hr | +9 |

| $R^1$ | $R^2$ | mg/kg p.o. | n | Blood Pressure: Pre-treat. MABP mm Hg | Blood Pressure: Max Δ mm Hg | Blood Pressure: Max Δ BP % | Heart Rate: Pre-treat. HR beats/min. | Heart Rate: Max Δ beats | Heart Rate: Max Δ HR % |
|---|---|---|---|---|---|---|---|---|---|
| | | 0.25 | 10 | 172 | -49 at 1Hr | -29 | 378 | +65 at 1Hr | +17 |
| | | | | | -48 at 5Hr | -28 | | | |
| | | 0.5 | 8 | 180 | -75 at 45min | -42 | 402 | +75 at 45min | +19 |
| | | | | | -75 at 5Hr | -42 | | +49 at 5Hr | +12 |
| $CF_3O$- | 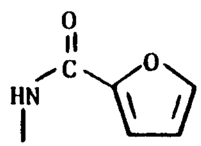 | 10 | 4 | 176 | -96 at 30min | -55 | 423 | +57 at 30min | +13 |
| | Control | 0 | 6 | 172 | -5 at 3Hr | -3 | 372 | +21 at 4Hr | +6 |

Compounds of formula (I) may be administered alone or with a diuretic, such as hydrochlorothianzide, or a $\beta$-blocker, such as propranolol or cetamolol in a suitable unit dose form.

## Claims

**Claims for the following Contracting States : AT, BE, CH, DE, FR, IT, LI, LU, NL, SE.**

**1.** A compound of formula (I)

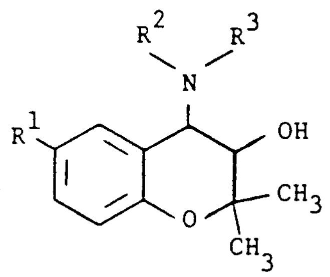

(I)

wherein $R^1$ is trifluoromethoxy or β, β, β-trifluoroethoxy; $R^2$ is selected from hydrogen, lower alkyl containing 1 to 5 carbon atoms, cyclo lower alkyl containing 5 to 8 carbon atoms,

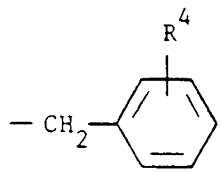 , 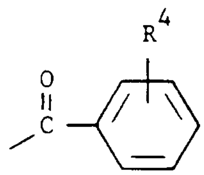 , 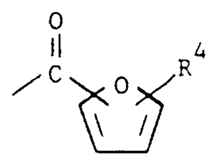 ;

$R^3$ is

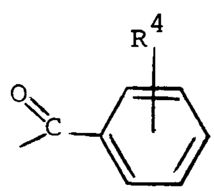 or 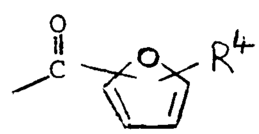

or $R^2$ and $R^3$ are joined together to form

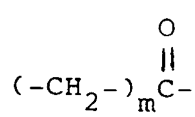

wherein m is 3 to 6; or $R^2$ and $R^3$ are joined together to form

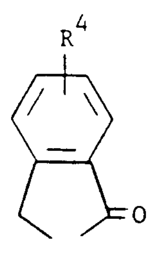 or 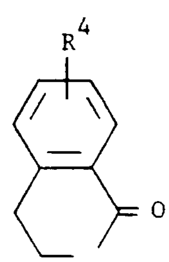

wherein $R^4$ is selected from the group consisting of hydrogen, alkoxy containing 1 to 5 carbon atoms, amino or mono- or disubstituted alkyl amino wherein said alkyl groups contain 1 to 5 carbon atoms and the pharmaceutically acceptable salt and solvates thereof

**2.** A compound according to Claim 1 in which $R^1$ is trifluoromethoxy.

**3.** A compounds according to Claim 1 or Claim 2 wherein $R^2$ is hydrogen and $R^3$ is

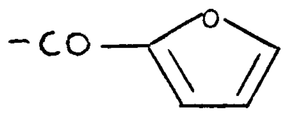

or $R^2$ and $R^3$ together represent

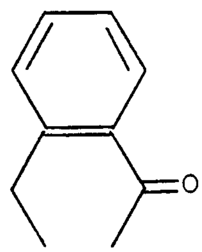

.

**4.** Trans-2-[2,3-dihydro-2,2-dimethyl-3-hydroxy-6-(trifluoromethoxy)-4H-I-benzopyran-4-yl]-2-3-dihydro-1H-isoindol-I-one and the pharmaceutically acceptable salts thereof.

**5.** Trans-N-[2,3-dihydro-2,2-dimethyl-3-hydroxy-6-(trifluoromethoxy)-4H-I-benzopyran-4-yl]-2-furancarboxamide and the pharmaceutically acceptable salts thereof.

**6.** (-)-3S,4R-Trans-2-[2,3-dihydro-2,2-dimethyl-3-hydroxy-6-(trifluoromethoxy)-4H-I-benzopyran-4-yl]-2,3-dihydro-IH-isoindol-I-one and the pharmaceutically acceptable salts thereof.

**7.** A basic compound as claimed in Claim 1 when in the form of a pharmaceutically acceptable acid addition salt.

**8.** A process for the preparation of a compound of formula I as defined in claim 1 which comprises
(a) reacting a compound of formula

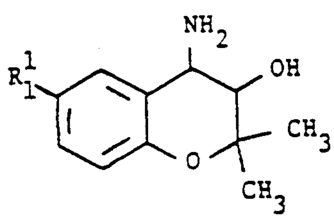

(II)

wherein $R_1^{\,1}$ is $R^1$ as defined in Claim 1 or a group or atom convertible thereto, with a compound of formula (III)

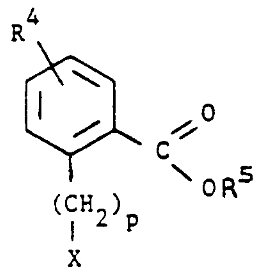

(III)

wherein X is chlorine, bromine, or iodine; $R^4$ is in Claim 1; p is I or 2 -$COOR^5$ is an ester function and if required converting the the $R_1^1$ group or atom to a group as defined by $R^1$ above to give a compound of formula I wherein $R^2$ and $R^3$ are joined together to form

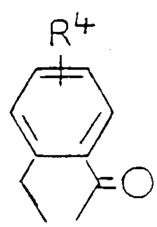

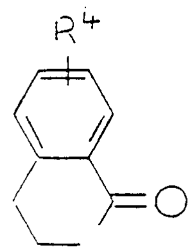

or

(b) reacting a compound of formula

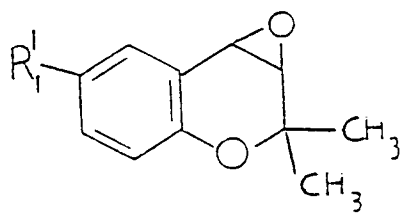

(IV)

wherein $R_1^1$ is as defined above, with a compound of formula:

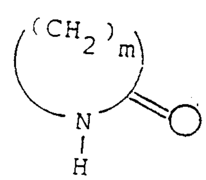

(VIII)

or

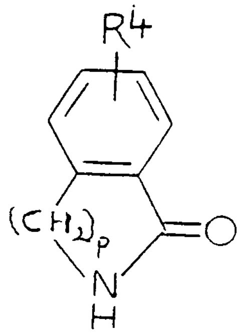

(IX)

or

$NH_2-(CH_2)_m-COOR^5$ (X)

in which formulae p is 1 or 2,
m is an integer from 3 to 6,
and $COOR^5$ is an ester function,
and if required converting the $R_1^1$ group or atom to a group as defined by $R^1$ above, to give a compound of formula I wherein $R^2$ and $R^3$ are joined to form

$-(CH_2)_mCO-$

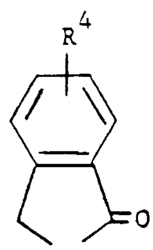

or

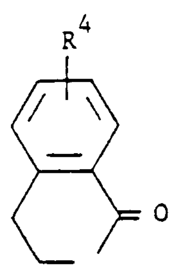

or
(c) acylating a compound of formula

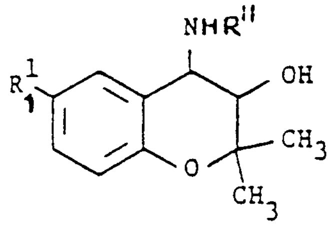

(XI)

wherein $R_1^1$ is as defined above and $R^{11}$ is hydrogen, $C_1$-$C_5$ alkyl, $C_5$-$C_8$ cycloalkyl,

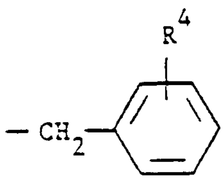 , 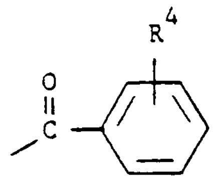 or 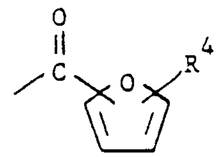 ;

with an acylating agent containing the group $R^{12}$ wherein $R^{12}$ is

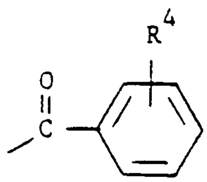 or 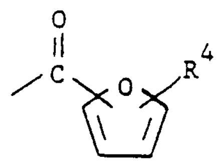

in which formulae $R^4$ is as defined above, and if required converting the $R_1^1$ group or atom to a group as defined by $R^1$ to give a compound of formula I wherein $R^2$ is $R^{11}$ as defined above and $R^3$ is $R^{12}$ as defined above;

or

(d) reacting a compound of formula II as defined above under reductive alkylation conditions with a compound of formula

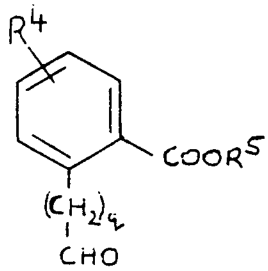 (XII)

wherein q is 0 or 1, $R^4$ and $R^5$ are as defined above and if required converting the $R_1^1$ group to $R^1$ to give a compound of formula I wherein $R^2$ and $R^3$ are joined together to form

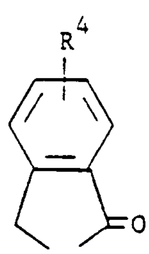 or 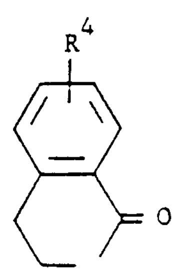

or

(e) reacting a compound of formula

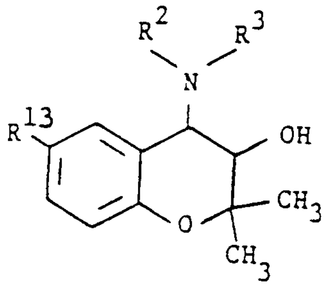

(XIII)

wherein $R^2$ and $R^3$ are as defined above and $R^{13}$ is a group or atom that can be converted directly to $R^1$, with a compound that converts $R^{13}$ to $R^1$,
or
(f) converting a basic compound of formula I to a pharmaceutically acceptable salt thereof by treatment with an acid or neutralising an acid addition salt with a base to give a compound of formula I in free base form.

**9.** A process for resolution of a racemate having formula I as defined in Claim 1 which comprises;
a) reacting said racemate of formula (I) with an optically pure chiral auxiliary, to form a mixture of two diastereomers;
b) separating said diastereomers by physical means, and
c) removing the chiral auxiliary from one or both diastereomers to afford one or both enantiomers.

**10.** A process as claimed in Claim 9 in which the chiral auxiliary is 1-(1-naphthyl)ethyl isocyanate or α-methylbenzyl isocyanate.

**11.** A pharmaceutical composition comprising a compound according to anyone of claims 1 to 6 or a pharmaceutically acceptable salt or solvate thereof and a pharmaceutically acceptable carrier.

**12.** A compound of formula I as claimed in any one of Claims 1 to 7 for use as a pharmaceutical.

**13.** A compound of formula II as shown in Claim 8 process (a) wherein $R^1$ is $CF_3O$- or $CF_3CH_2O$-.

**14.** A compound of formula IV as shown in Claim 8 process (b) wherein $R^1$ is $CF_3O$- or $CF_3CH_2O$-.

**Claims for the following Contracting States : ES, GR.**

**1.** A process for preparing a compound of formula I

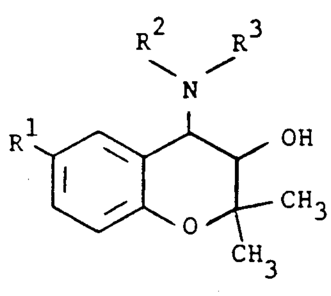

(I)

wherein $R^1$ is trifluoromethoxy or β, β, β-trifluoroethoxy; $R^2$ is selected from hydrogen, lower alkyl containing l to 5 carbon atoms, cyclo lower alkyl containing 5 to 8 carbon atoms,

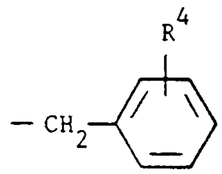 , 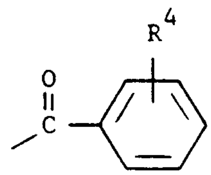 , 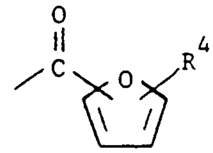 ;

$R^3$ is

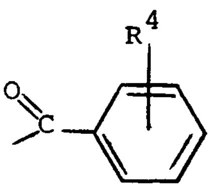 or 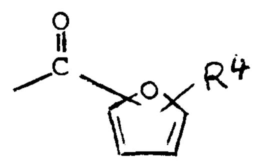

or $R^2$ and $R^3$ are joined together to form

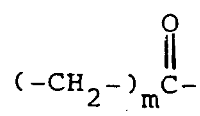

wherein m is 3 to 6; or $R^2$ and $R^3$ are joined together to form

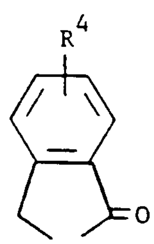 or 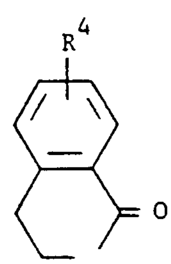

wherein $R^4$ is selected from the group consisting of hydrogen, alkoxy containing l to 5 carbon atoms, amino or mono- or disubstituted alkyl amino wherein said alkyl groups contain 1 to 5 carbon atoms or a pharmaceutically acceptable salt or solvate thereof which comprises

(a) reacting a compound of formula

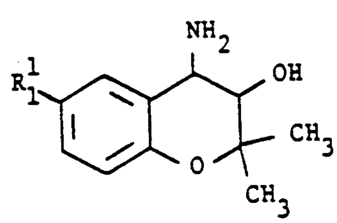

(II)

wherein $R_1^1$ is $R^1$ as defined above or a group or atom convertible thereto, with a compound of formula (III)

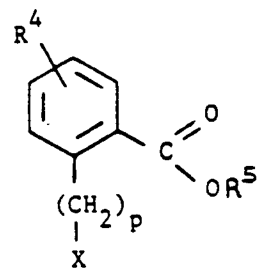

(III)

wherein X is chlorine, bromine, or iodine; $R^4$ is above p is I or 2 -$COOR^5$ is an ester function and if required converting the the $R_1^1$ group or atom to a group as defined by $R^1$ above to give a compound of formula I wherein $R^2$ and $R^3$ are joined together, to form

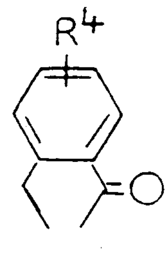

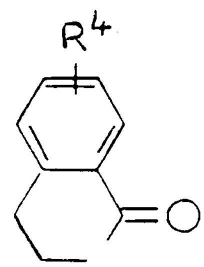

or

(b) reacting a compound of formula

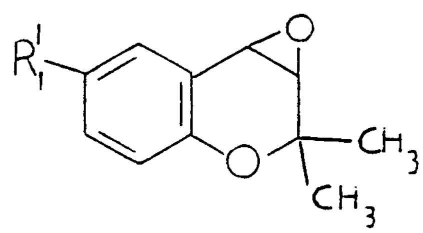

(IV)

wherein $R_1^1$ is as defined above, with a compound of formula:

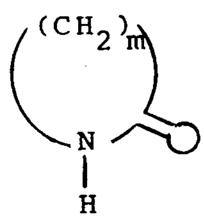

(VIII)

or

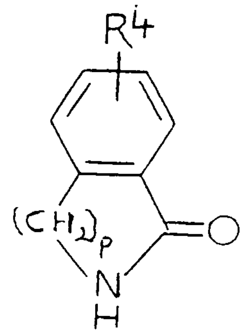

(IX)

or

$NH_2-(CH_2)_m-COOR^5$ (X)

in which formulae p is l or 2,
m is an integer from 3 to 6,
and $COOR^5$ is an ester function,
and if required converting the $R_1^1$ group or atom to a group as defined by $R^1$ above, to give a compound of formula I wherein $R^2$ and $R^3$ are joined to form

$-(CH_2)_mCO-$

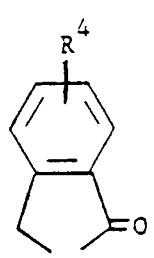

or

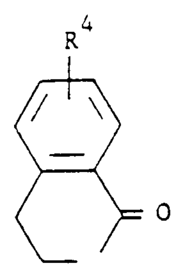

or
(c) acylating a compound of formula

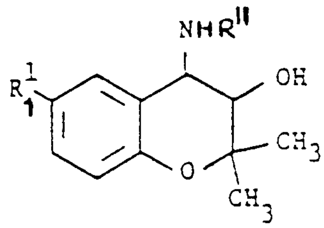

(XI)

wherein $R_1^1$ is as defined above and $R^{11}$ is hydrogen, $C_1$-$C_5$ alkyl, $C_5$-$C_8$ cycloalkyl,

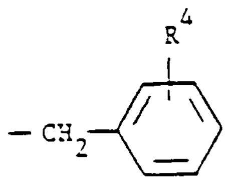
,
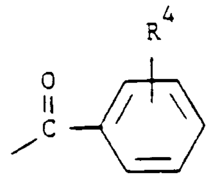
or
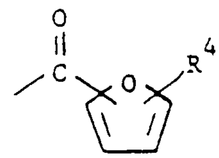
;

with an acylating agent containing the group $R^{12}$ wherein $R^{12}$ is

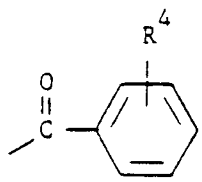
or
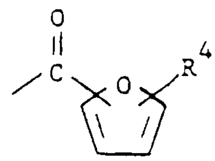

in which formulae $R^4$ is as defined above, and if required converting the $R^1_1$ group or atom to a group as defined by $R^1$ to give a compound of formula I wherein $R^2$ is $R^{11}$ as defined above and $R^3$ is $R^{12}$ as defined above;

or

(d) reacting a compound of formula II as defined above under reductive alkylation conditions with a compound of formula

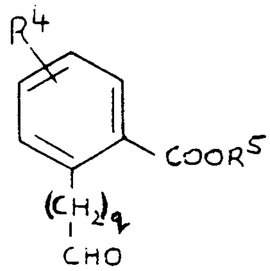
(XII)

wherein q is 0 or 1, $R^4$ and $R^5$ are as defined above and if required converting the $R^1_1$ group to $R^1$ to give a compound of formula I wherein $R^2$ and $R^3$ are joined together to form

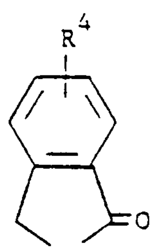
or
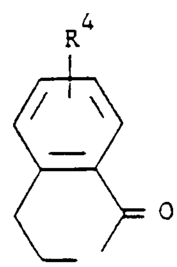

or

(e) reacting a compound of formula

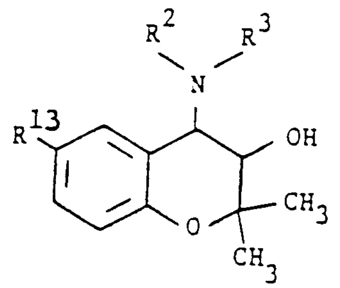

(XIII)

wherein $R^2$ and $R^3$ are as defined above and $R^{13}$ is a group or atom that can be converted directly to $R^1$, with a compound that converts $R^{13}$ to $R^1$,

or

(f) converting a basic compound of formula I to a pharmaceutically acceptable salt thereof by treatment with an acid or neutralising an acid addition salt with a base to give a compound of formula I in free base form.

**2.** A process according to Claim 1 in which $R^1$ is trifluoromethoxy.

**3.** A process according to Claim 1 or Claim 2 wherein $R^2$ is hydrogen and $R^3$ is

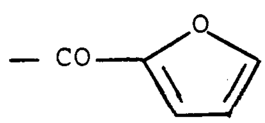

or $R^2$ and $R^3$ together represent

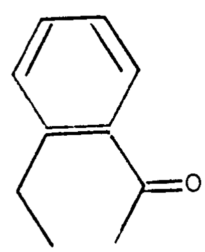

**4.** A process as claimed in Claim 1 in which the compound prepared is trans-2-[2,3-dihydro-2,2-dimethyl-3-hydroxy-6-(trifluoromethoxy)-4H-l-benzopyran-4-yl]-2,3-dihydro-1H-isoindol-l-one and the pharmaceutically acceptable salts thereof.

**5.** A process as claimed in Claim 1 in which the compound prepared is trans-N-[2,3-dihydro-2,2-dimethyl-3-hydroxy-6-(trifluoromethoxy)-4H-l-benzopyran-4-yl]-2-furancarboxamide and the pharmaceutically acceptable salts thereof.

**6.** A process as claimed in Claim 1 in which the compound prepared is (-)-3S,4R-trans-2-[2,3-dihydro-2,2-dimethyl-3-hydroxy-6-(trifluoromethoxy)-4H-l-benzopyran-4-yl]-2,3-dihydro-1H-isoindol-l-one and the pharmaceutically acceptable salts thereof.

**7.** A process for resolution of a racemate having formula I as defined in Claim 1 which comprises;

a) reacting said racemate of formula (I) with an optically pure chiral auxiliary, to form a mixture of two diastereomers;

b) separating said diastereomers by physical means, and
c) removing the chiral auxiliary from one or both diastereomers to afford one or both enantiomers.

**8.** A process as claimed in Claim 7 in which the chiral auxiliary is 1-(1-naphthyl)ethyl isocyanate or $\alpha$-methylbenzyl isocyanate.

**9.** A process for preparing a pharmaceutical composition comprising bringing a compound of formula I as defined in Claim 1 or a pharmaceutically acceptable salt or solvate thereof into association with a pharmaceutically acceptable carrier.

**10.** A compound of formula II as shown in Claim 1 process (a) wherein $R^1$ is $CF_3O$- or $CF_3CH_2O$-.

**11.** A compound of formula IV as shown in Claim 1 process (b) wherein $R^1$ is $CF_3O$- or $CF_3CH_2O$-.

**Patentansprüche**
**Patentansprüche für folgende Vertragsstaaten : AT, BE, CH, DE, FR, IT, LI, LU, NL, SE.**

**1.** Verbindung der Formel (I)

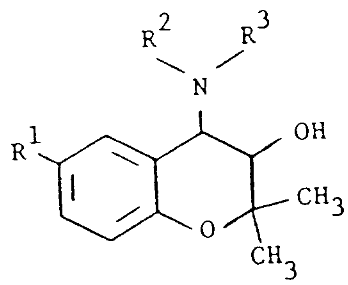

(I),

worin $R^1$ Trifluormethoxy oder $\beta,\beta,\beta$-Trifluorethoxy bedeutet, $R^2$ ausgewählt ist aus Wasserstoff, nied.Alkyl mit 1 bis 5 Kohlenstoffatomen, Cyclo-nied.alkyl mit 5 bis 8 Kohlenstoffatomen,

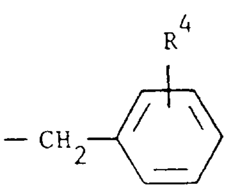 , 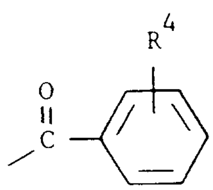 oder 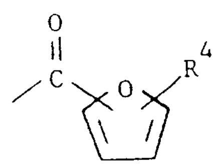 ;

$R^3$

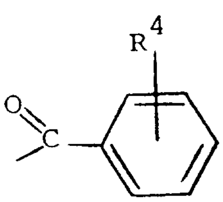 oder 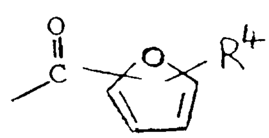

darstellt oder $R^2$ und $R^3$ unter Bildung von

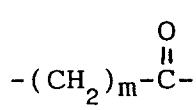

miteinander verbunden sind, wobei m 3 bis 6 ist; oder $R^2$ und $R^3$ unter Bildung von

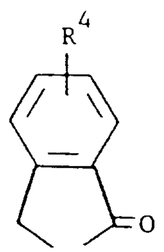

oder

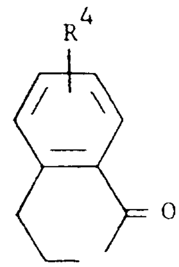

miteinander verbunden sind, wobei $R^4$ ausgewählt ist aus der Gruppe bestehend aus Wasserstoff, Alkoxy mit 1 bis 5 Kohlenstoffatomen, Amino oder mono- oder disubstituiertem Alkylamino, wobei die Alkylgruppen 1 bis 5 Kohlenstoffatome enthalten, und die pharmazeutisch annehmbaren Salze und Solvate hievon.

**2.** Verbindung nach Anspruch 1, in der $R^1$ Trifluormethoxy ist.

**3.** Verbindungen nach Anspruch 1 oder 2, in denen $R^2$ Wasserstoff ist und $R^3$

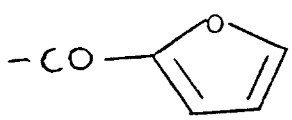

bedeutet oder $R^2$ und $R^3$ miteinander

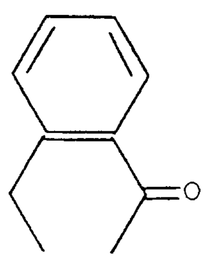

sind.

**4.** trans-2-[2,3-Dihydro-2,2-dimethyl-3-hydroxy-6-(trifluormethoxy)-4H-1-benzopyran-4-yl]-2,3-dihydro-1H-isoindol-1-on und die pharmazeutisch annehmbaren Salze hievon.

**5.** trans-N-[2,3-Dihydro-2,2-dimethyl-3-hydroxy-6-(trifluormethoxy)-4H-1-benzopyran-4-yl]-2-furancarboxamid und die pharmazeutisch annehmbaren Salze hievon.

**6.** (-)-3S,4R-trans-2-[2,3-Dihydro-2,2-dimethyl-3-hydroxy-6-(trifluormethoxy)-4H-1-benzopyran-4-yl]-2,3-dihydro-1H-isoindol-1-on und die pharmazeutisch annehmbaren Salze hievon.

**7.** Basische Verbindung nach Anspruch 1, wenn sie in Form eines pharmazeutisch annehmbaren Säureadditionssalzes ist.

**8.** Verfahren zur Herstellung einer Verbindung der Formel (I) nach Anspruch 1, das umfaßt:

(a) das Umsetzen einer Verbindung der Formel

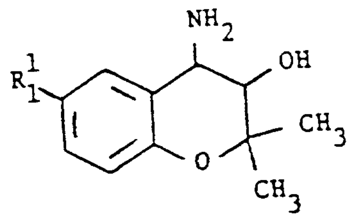

(II),

worin $R^1_1$ die Bedeutung $R^1$ wie oben definiert hat oder eine Gruppe oder ein Atom ist, die bzw. das in dieses überführbar ist, mit einer Verbindung der Formel (III)

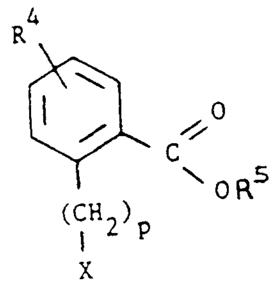

(III),

worin X Chlor, Brom oder Iod bedeutet; $R^4$ wie in Anspruch 1 definiert ist; p 1 oder 2 ist und $-COOR^5$ eine Esterfunktion darstellt, und, wenn erforderlich, das Überführen der Gruppe oder des Atoms $R^1_1$ in eine Gruppe, wie oben durch $R^1$ definiert, wobei eine Verbindung der Formel (I) erhalten wird, worin $R^2$ und $R^3$ unter Bildung von

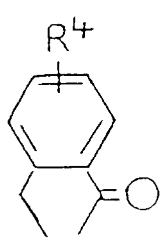

oder

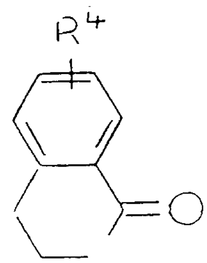

miteinander verbunden sind, oder
(b) das Umsetzen einer Verbindung der Formel

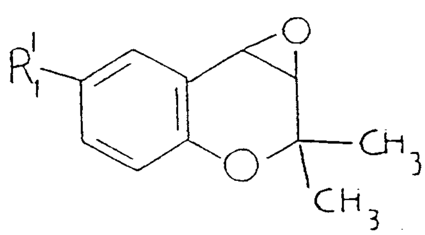

(IV),

worin $R^1_1$ wie oben definiert ist, mit einer Verbindung der Formel

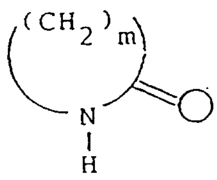

(VIII)

,

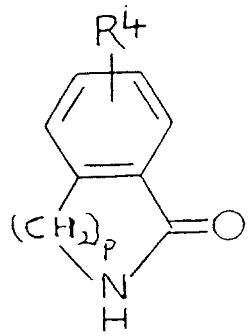

(IX)

oder

$NH_2-(CH_2)_m-COOR^5$ (X),

in welchen Formeln p 1 oder 2 ist, m eine ganze Zahl von 3 bis 6 ist und $COOR^5$ eine Esterfunktion darstellt, und, wenn erforderlich, das Überführen der Gruppe oder des Atoms $R^1{}_1$ in eine Gruppe, wie oben durch $R^1$ definiert, wobei eine Verbindung der Formel (I) erhalten wird, worin $R^2$ und $R^3$ unter Bildung von

$-(CH_2)_mCO-$,

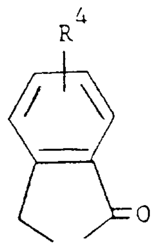

oder

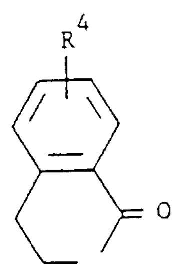

verbunden sind, oder
(c) das Acylieren einer Verbindung der Formel

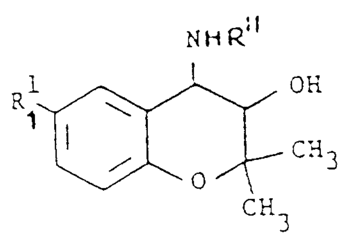

(XI),

worin $R^1{}_1$ wie oben definiert ist und $R^{11}$ Wasserstoff, $C_1$-$C_5$-Alkyl, $C_5$-$C_8$-Cycloalkyl,

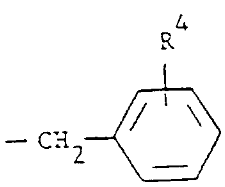

,

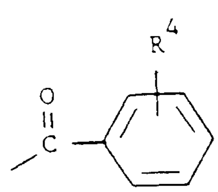

oder

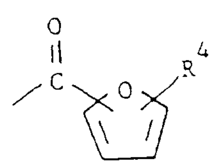

darstellt, mit einem die Gruppe $R^{12}$ enthaltenden Acylierungsmittel, wobei $R^{12}$

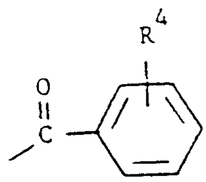

oder

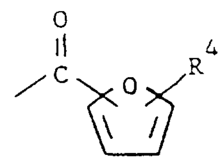

bedeutet, in welchen Formeln $R^4$ wie oben definiert ist, und, wenn erforderlich, das Überführen der Gruppe oder des Atoms $R^1{}_1$ in eine Gruppe, wie durch $R^1$ definiert, wobei eine Verbindung der Formel (I) erhalten wird, worin $R^2$ die Bedeutung $R^{11}$ wie oben definiert hat und $R^3$ die Bedeutung $R^{12}$ wie oben definiert hat, oder

(d) das Umsetzen einer Verbindung der Formel (II), wie oben definiert, unter reduktiven Alkylierungsbedingungen mit einer Verbindung der Formel

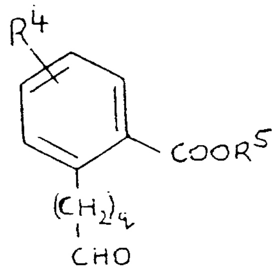

(XII),

worin q Null oder 1 ist und $R^4$ und $R^5$ wie oben definiert sind, und, wenn erforderlich, das Überführen der Gruppe oder des Atoms $R^1{}_1$ in eine Gruppe $R^1$, wobei eine Verbindung der Formel (I) erhalten wird, worin $R^2$ und $R^3$ unter Bildung von

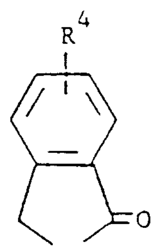

oder

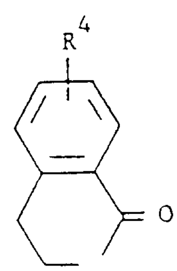

miteinander verbunden sind, oder

(e) das Umsetzen einer Verbindung der Formel

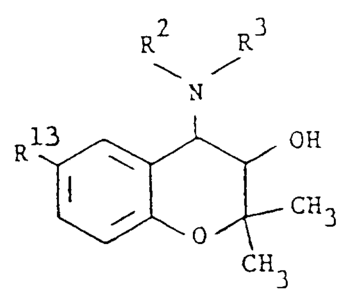

(XIII),

worin $R^2$ und $R^3$ wie oben definiert sind und $R^{13}$ eine Gruppe oder ein Atom ist, die bzw. das direkt in $R^1$ überführt werden kann, mit einer Verbindung, die $R^{13}$ in $R^1$ überführt, oder

(f) das Überführen einer basischen Verbindung der Formel (I) in ein pharmazeutisch annehmbares Salz hievon durch Behandlung mit einer Säure oder das Neutralisieren eines Säureadditionssalzes mit einer Base, wobei eine Verbindung der Formel (I) in Form der freien Base erhalten wird.

9. Verfahren zum Trennen eines Racemats der Formel (I), wie in Anspruch 1 definiert, das umfaßt
(a) das Umsetzen des Racemats der Formel (I) mit einem optisch reinen chiralen Hilfsmittel unter Bildung einer Mischung von zwei Diastereomeren,
(b) das Trennen der Diastereomere auf physikalischem Weg und
(c) das Entfernen des chiralen Hilfsmittels aus einem oder beiden Diastereomeren, um ein oder beide Enantiomere zu erhalten.

10. Verfahren nach Anspruch 9, in dem das chirale Hilfsmittel 1-(1-Naphthyl)-ethylisocyanat oder $\alpha$-Methylbenzylisocyanat ist.

11. Pharmazeutische Zusammensetzung umfassend eine Verbindung nach einem der Ansprüche 1 bis 6 oder ein pharmazeutisch annehmbares Salz oder Solvat hievon und einen pharmazeutisch annehmbaren Träger.

12. Verbindung nach Anspruch (I) nach einem der Ansprüche 1 bis 7 zur Verwendung als Pharmazeutikum.

13. Verbindung der Formel (II), wie in Anspruch 8, Verfahren (a) gezeigt, in der $R^1$ $CF_3O$- oder $CF_3CH_2O$-ist.

14. Verbindung der Formel (IV), wie in Anspruch 8, Verfahren (b) gezeigt, in der $R^1$ $CF_3O$- oder $CF_3CH_2O$-ist.

**Patentansprüche für folgende Vertragsstaaten : ES, GR.**

1. Verfahren zum Herstellen einer Verbindung der Formel (I)

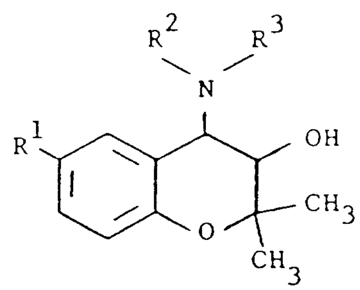

(I),

worin $R^1$ Trifluormethoxy oder $\beta,\beta,\beta$-Trifluorethoxy bedeutet, $R^2$ ausgewählt ist aus Wasserstoff, nied.Alkyl mit 1 bis 5 Kohlenstoffatomen, Cyclo-nied.alkyl mit 5 bis 8 Kohlenstoffatomen,

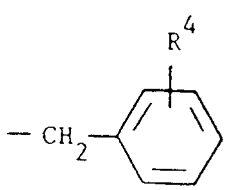

,

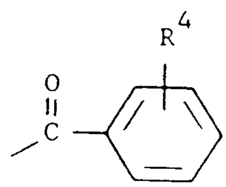

oder

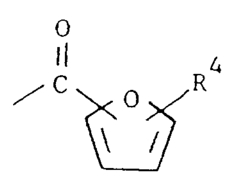

;

$R^3$

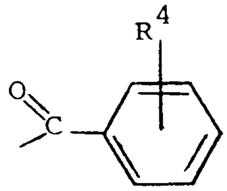

oder

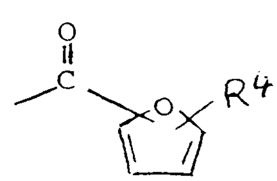

darstellt oder $R^2$ und $R^3$ unter Bildung von

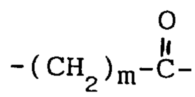

miteinander verbunden sind, wobei m 3 bis 6 ist; oder $R^2$ und $R^3$ unter Bildung von

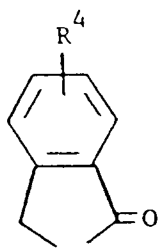

oder

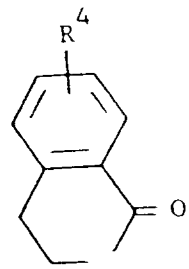

miteinander verbunden sind, wobei $R^4$ ausgewählt ist aus der Gruppe bestehend aus Wasserstoff, Alkoxy mit 1 bis 5 Kohlenstoffatomen, Amino oder mono- oder disubstituiertem Alkylamino, wobei die Alkylgruppen 1 bis 5 Kohlenstoffatome enthalten, oder eines pharmazeutisch annehmbaren Salzes oder Solvates hievon, welches umfaßt

(a) das Umsetzen einer Verbindung der Formel

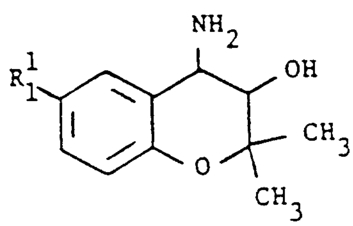

(II),

worin $R^1_1$ die Bedeutung $R^1$ wie oben definiert hat oder eine Gruppe oder ein Atom ist, die bzw. das in dieses überführbar ist, mit einer Verbindung der Formel (III)

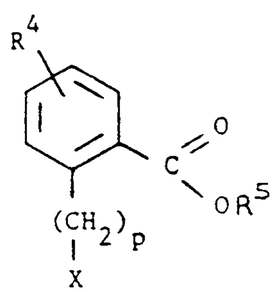

(III),

worin X Chlor, Brom oder Iod bedeutet; $R^4$ wie oben definiert ist; p 1 oder 2 ist und $-COOR^5$ eine Esterfunktion darstellt, und, wenn erforderlich, das Überführen der Gruppe oder des Atoms $R^1_1$ in eine Gruppe, wie oben durch $R^1$ definiert, wobei eine Verbindung der Formel (I) erhalten wird, worin $R^2$ und $R^3$ unter Bildung von

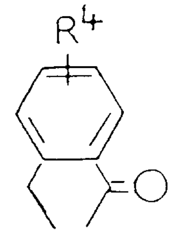

oder

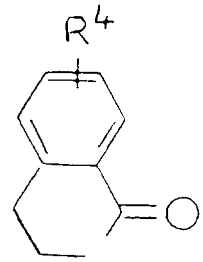

miteinander verbunden sind, oder

(b) das Umsetzen einer Verbindung der Formel

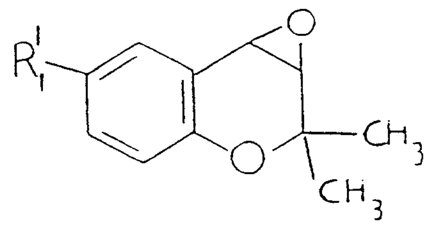

(IV),

worin $R^1{}_1$ wie oben definiert ist, mit einer Verbindung der Formel

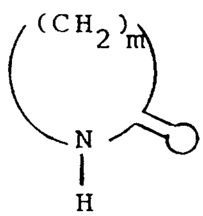

(VIII)

,

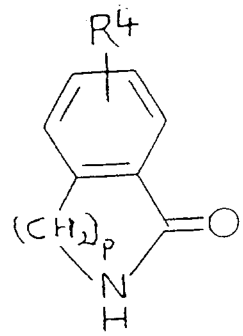

(IX)

oder

$NH_2$-$(CH_2)_m$-$COOR^5$ (X),

in welchen Formeln p 1 oder 2 ist, m eine ganze Zahl von 3 bis 6 ist und $COOR^5$ eine Esterfunktion darstellt, und, wenn erforderlich, das Überführen der Gruppe oder des Atoms $R^1{}_1$ in eine Gruppe, wie oben durch $R^1$ definiert, wobei eine Verbindung der Formel (I) erhalten wird, worin $R^2$ und $R^3$ unter Bildung von

-$(CH_2)_m$CO-,

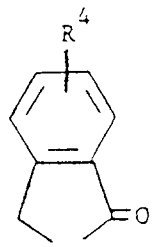

oder

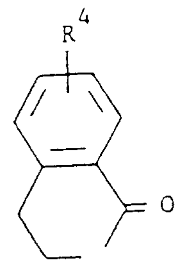

verbunden sind, oder

(c) das Acylieren einer Verbindung der Formel

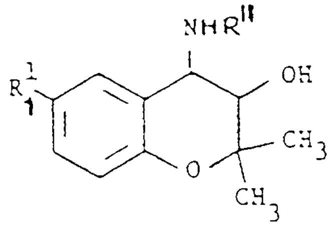

(XI),

worin $R^1{}_1$ wie oben definiert ist und $R^{11}$ Wasserstoff, $C_1$-$C_5$-Alkyl, $C_5$-$C_8$-Cycloalkyl,

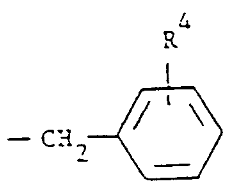

,

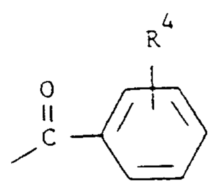

oder

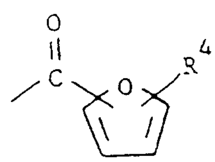

darstellt, mit einem die Gruppe $R^{12}$ enthaltenden Acylierungsmittel, wobei $R^{12}$

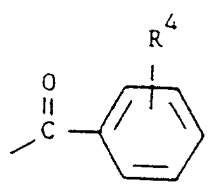

oder

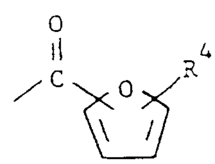

bedeutet, in welchen Formeln $R^4$ wie oben definiert ist, und, wenn erforderlich, das Überführen der Gruppe oder des Atoms $R^1{}_1$ in eine Gruppe, wie durch $R^1$ definiert, wobei eine Verbindung der Formel (1) erhalten wird, worin $R^2$ die Bedeutung $R^{11}$ wie oben definiert hat und $R^3$ die Bedeutung $R^{12}$ wie oben definiert hat, oder

(d) das Umsetzen einer Verbindung der Formel (II), wie oben definiert, unter reduktiven Alkylierungsbedingungen mit einer Verbindung der Formel

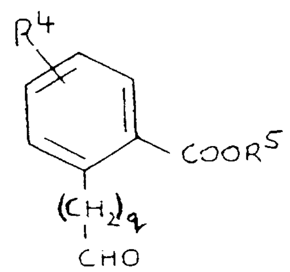

(XII),

worin g Null oder 1 ist und $R^4$ und $R^5$ wie oben definiert sind, und, wenn erforderlich, das Überführen der Gruppe oder des Atoms $R^{1}_{1}$ in eine Gruppe $R^1$, wobei eine Verbindung der Formel (I) erhalten wird, worin $R^2$ und $R^3$ unter Bildung von

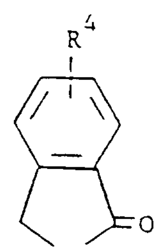

oder

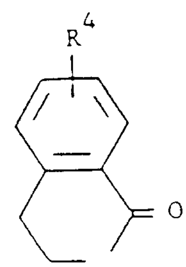

miteinander verbunden sind, oder
(e) das Umsetzen einer Verbindung der Formel

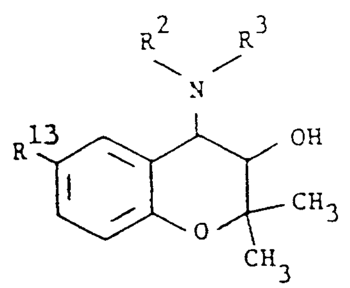

(XIII),

worin $R^2$ und $R^3$ wie oben definiert sind und $R^{13}$ eine Gruppe oder ein Atom ist, die bzw. das direkt in $R^1$ überführt werden kann, mit einer Verbindung, die $R^{13}$ in $R^1$ überführt, oder
(f) das Überführen einer basischen Verbindung der Formel (I) in ein pharmazeutisch annehmbares Salz hievon durch Behandlung mit einer Säure oder das Neutralisieren eines Säureadditionssalzes mit einer Base, wobei eine Verbindung der Formel (I) in Form der freien Base erhalten wird.

**2.** Verfahren nach Anspruch 1, wobei $R^1$ Trifluormethoxy ist.

**3.** Verfahren nach Anspruch 1 oder 2, wobei $R^2$ Wasserstoff ist und $R^3$

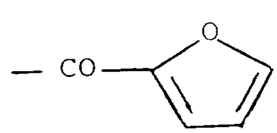

bedeutet oder $R^2$ und $R^3$ miteinander

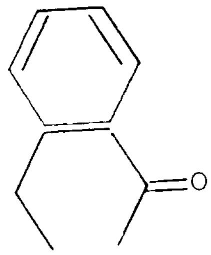

sind.

**4.** Verfahren nach Anspruch 1, in dem die hergestellte Verbindung trans-2-[2,3-Dihydro-2,2-dimethyl-3-hydroxy-6-(trifluormethoxy)-4H-1-benzopyran-4-yl]-2,3-dihydro-1H-isoindol-1-on und die pharmazeutisch annehmbaren Salze hievon ist.

**5.** Verfahren nach Anspruch 1, in dem die hergestellte Verbindung trans-N-[2,3-Dihydro-2,2-dimethyl-3-hydroxy-6-(trifluormethoxy)-4H-1-benzopyran-4-yl]-2-furancarboxamid und die pharmazeutisch annehmbaren Salze hievon ist.

**6.** Verfahren nach Anspruch 1, in dem die hergestellte Verbindung (-)-3S,4R-trans-2-[2,3-Dihydro-2,2-dimethyl-3-hydroxy-6-(trifluormethoxy)-4H-1-benzopyran-4-yl]-2,3-dihydro-1H-isoindol-1-on und die pharmazeutisch annehmbaren Salze hievon ist.

**7.** Verfahren zum Trennen eines Racemats der Formel (I), wie in Anspruch 1 definiert, das umfaßt
(a) das Umsetzen des Racemats der Formel (I) mit einem optisch reinen chiralen Hilfsmittel unter Bildung einer Mischung von zwei Diastereomeren,
(b) das Trennen der Diastereomere auf physikalischem Weg und
(c) das Entfernen des chiralen Hilfsmittels aus einem oder beiden Diastereomeren, um ein oder beide Enantiomere zu erhalten.

**8.** Verfahren nach Anspruch 7, in dem das chirale Hilfsmittel 1-(1-Naphthyl)-ethylisocyanat oder $\alpha$-Methylbenzylisocyanat ist.

**9.** Verfahren zum Herstellen einer pharmazeutischen Zusammensetzung, das das Vereinigen einer Verbindung der Formel (I), wie in Anspruch 1 definiert, oder eines pharmazeutisch annehmbaren Salzes oder Solvates hievon mit einem pharmazeutisch annehmbaren Träger umfaßt.

**10.** Verbindung der Formel (II), wie in Anspruch 1, Verfahren (a) gezeigt, in der $R^1$ $CF_3O$- oder $CF_3CH_2O$-ist.

**11.** Verbindung der Formel (IV), wie in Anspruch 1, Verfahren (b) gezeigt, in der $R^1$ $CF_3O$- oder $CF_3CH_2O$-ist.

## Revendications

**Revendications pour les Etats contractants suivants : AT, BE, CH, DE, FR, IT, LI, LU, NL, SE.**

**1.** Composé de formule (I)

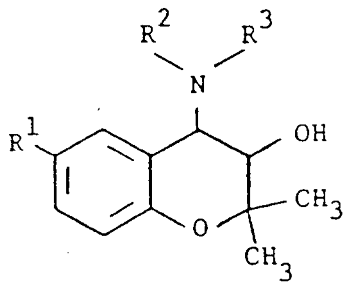

(I)

où $R^1$ est trifluorométhoxy ou $\beta,\beta,\beta$-trifluoroéthoxy; $R^2$ est choisi parmi hydrogène, alcoyle inférieur contenant de 1 à 5 atomes de carbone, cycloalcoyle inférieur contenant de 5 à 8 atomes de Carbone,

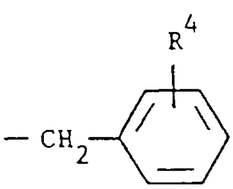 , 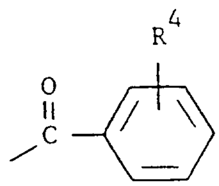 , 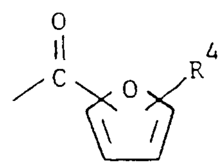 ;

$R^3$ est

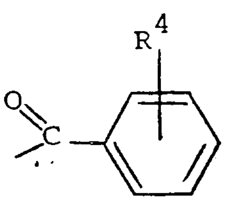 ou 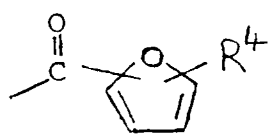

ou $R^2$ et $R^3$ sont liés ensemble pour former $-(CH_2-)_mCO-$ où m est 3 à 6,
ou $R^2$ et $R^3$ sont liés ensemble pour former

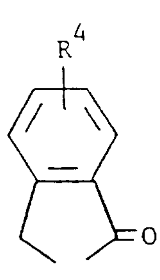 ou 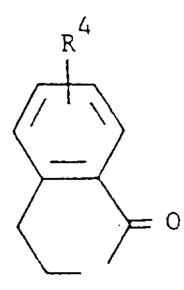

où $R^4$ est choisi parmi le groupe consistant en hydrogène, alcoxy contenant de 1 à 5 atomes de carbone, amino ou amino mono- ou disubstitué par un alcoyle, ces groupes alcoyle contenant de 1 à 5 atomes de carbone, et les sels et dérivés solvatés pharmaceutiquement acceptables de celui-ci.

**2.** Composé selon la revendication 1, dans lequel $R^1$ est trifluorométhoxy.

**3.** Composé selon la revendication 1 ou 2 dans lequel $R^2$ est hydrogène et $R^3$ est

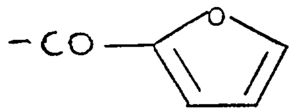

ou $R^2$ et $R^3$ représentent ensemble

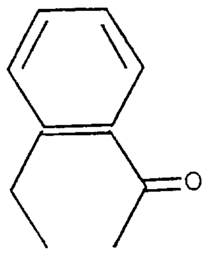

.

**4.** La trans-2-[2,3-dihydro-2,2-diméthyl-3-hydroxy-6-(trifluorométhoxy)-4H-1-benzopyran-4-yl]-2,3-dihydro-1H-isoindol-1-one et les sels pharmaceutiquement acceptables de celle-ci.

**5.** Le trans-N-[2,3-dihydro-2,2-diméthyl-3-hydroxy-6-(trifluorométhoxy)-4H-1-benzopyran-4-yl)-2-furancarboxamide et les sels pharmaceutiquement acceptables de celui-ci.

**6.** La (-)-3S,4R-trans-2-[2,3-dihydro-2,2-diméthyl-3-hydroxy-6-(trifluorométhoxy)-4H-1-benzopyran-4-yl)-2,3-dihydro-1H-isoindol-1-one et les sels pharmaceutiquement acceptables de celle-ci.

**7.** Composé basique selon la revendication 1, sous la forme d'un sel d'addition d'acide pharmaceutiquement acceptable.

**8.** Procédé pour la préparation d'un composé de formule (I) tel que défini dans la revendication 1, qui comprend :

a) la réaction d'un composé de formule

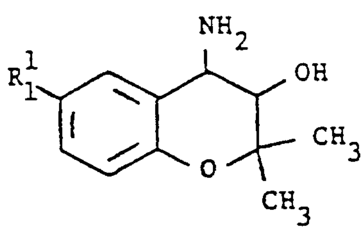

(II)

où $R_1{}^1$ est $R^1$ tel que défini dans la revendication 1 ou un groupe ou un atome convertible en celui-ci, avec un composé de formule (III)

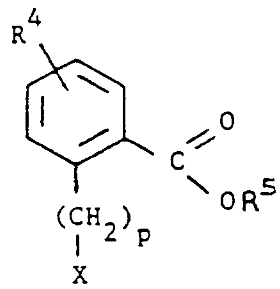

(III)

où X est chlore, brome ou iode; $R^4$ est tel que défini dans la revendication 1; p est 1 ou 2; $-COOR^5$ est une fonction ester;

et, si nécessaire, la conversion du groupe ou de l'atome $R_1{}^1$ en un groupe tel que défini par $R^1$ ci-dessus, pour donner un composé de formule (I) ou $R^2$ et $R^3$ sont liés ensemble pour former

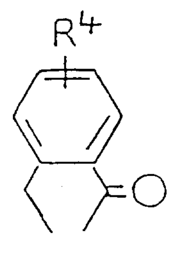

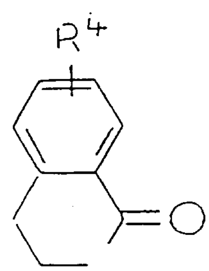

ou

b) la réaction d'un composé de formule

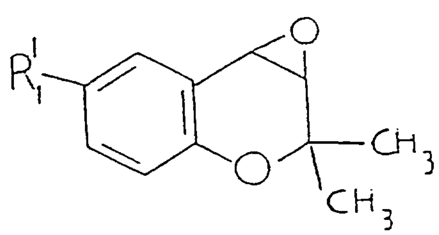

(IV)

où $R_1{}^1$ est tel que défini ci-dessus, avec un composé de formule

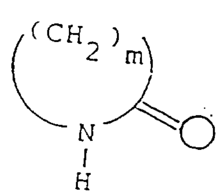

(VIII)

ou

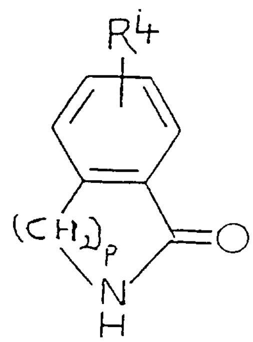

(IX)

ou

$NH_2-(CH_2)_m-COOR^5$ (X)

formules dans lesquelles p est 1 ou 2, m est un entier de 3 à 6, et, $-COOR^5$ est une fonction ester,

et, si nécessaire, la conversion du groupe ou de l'atome $R_1{}^1$ en un groupe tel que défini par $R^1$ ci-dessus, pour donner un composé de formule (I) où $R^2$ et $R^3$ sont liés ensemble pour former

$-(CH_2)_m-CO-$

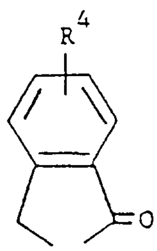

ou

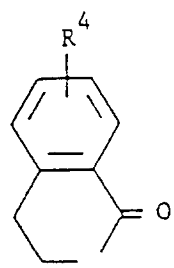

ou

c) l'acylation d'un composé de formule

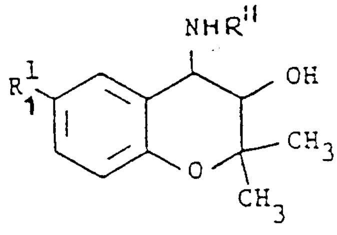

(XI)

où $R_1{}^1$ est tel que défini ci-dessus et $R^{11}$ est hydrogène, alcoyle en $C_1$-$C_5$, cycloalcoyle en $C_5$-$C_8$,

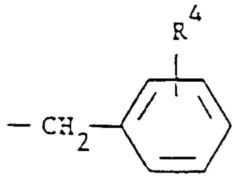

,

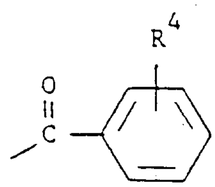

ou

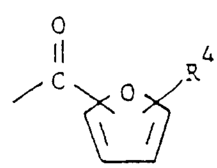

;

avec un agent acylant contenant le groupe $R^{12}$, où $R^{12}$ est

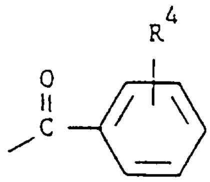

ou

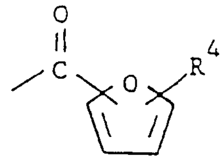

formules dans lesquelles $R^4$ est tel que défini ci-dessus,

et, si nécessaire, la conversion du groupe ou de l'atome $R_1^1$ en un groupe tel que défini par $R^1$ pour donner un composé de formule (I) où $R^2$ est $R^{11}$ tel que défini ci-dessus et $R^3$ est $R^{12}$ tel que défini ci-dessus;

ou

d) la réaction d'un composé de formule (II) tel que défini ci-dessus sous des conditions d'alcoylation réductrices avec un composé de formule

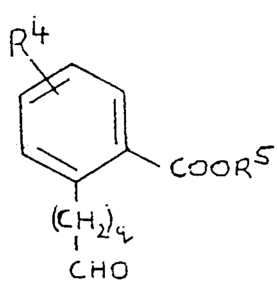

(XII)

où q est 0 ou 1, $R^4$ et $R^5$ sont tels que définis ci-dessus

et, si nécessaire, la conversion du groupe $R_1^1$ en $R^1$ pour donner un composé de formule (I) où $R^2$ et $R^3$ sont liés ensemble pour former

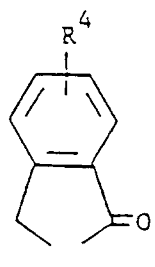

ou

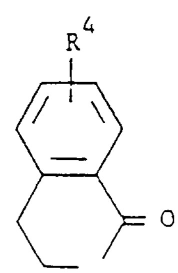

ou

e) la réaction d'un composé de formule

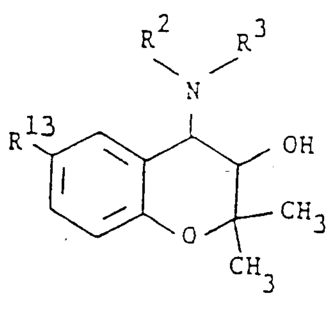

(XIII)

où $R^2$ et $R^3$ sont tels que définis ci-dessus, et $R^{13}$ est un groupe ou un atome qui peut être converti directement en $R^1$, avec un composé qui convertit $R^{13}$ en $R^1$,

ou

f) la conversion d'un composé basique de formule (I) en un sel pharmaceutiquement acceptable de celui-ci par traitement avec un acide ou la neutralisation d'un sel d'addition d'acide avec une base pour donner un composé de formule (I) sous forme de base libre.

**9.** Procédé pour la résolution d'un racémique de formule (I) tel que défini dans la revendication 1, qui comprend:

a) la réaction de ce racémique de formule (I) avec un auxiliaire chiral optiquement pur, pour former un mélange de deux diastéréoisomères;

b) la séparation de ces diastéréoisomères par des moyens physiques, et

c) l'élimination de l'auxiliaire chiral de l'un des diastéréoisomères ou des deux pour obtenir l'un des énantiomères ou les deux.

**10.** Un procédé selon la revendication 9, dans lequel l'auxiliaire chiral est de l'isocyanate de 1-(1-naphtyl)-éthyle ou l'isocyanate d'$\alpha$-méthylbenzyle.

**11.** Composition pharmaceutique comprenant un composé selon l'une quelconque des revendications 1 à 6, ou un sel ou dérivé solvaté pharmaceutiquement acceptable de celui-ci, et un excipient pharmaceutiquement acceptable.

**12.** Composé de formule (I) selon l'une quelconque des revendications 1 à 7 à utiliser en tant que produit pharmaceutique.

**13.** Composé de formule (II) tel que représenté dans la revendication 8, procédé a), où $R^1$ est $CF_3O$- ou $CF_3CH_2O$-.

**14.** Composé de formule (IV) tel que représenté dans la revendication 8, procédé b), où $R^1$ est $CF_3O$- ou $CF_3CH_2O$-.

**Revendications pour les Etats contractants suivants : ES, GR.**

**1.** Procédé pour la préparation d'un composé de formule (I)

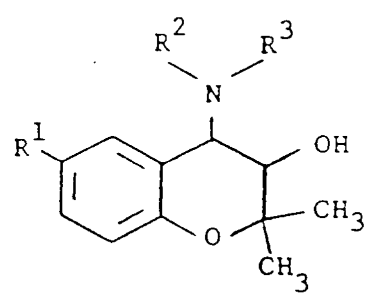

(I)

où $R^1$ est trifluorométhoxy ou un $\beta,\beta,\beta$-trifluoroéthoxy; $R^2$ est choisi parmi hydrogène, alcoyle inférieur contenant de 1 à 5 atomes de carbone, cycloalcoyle inférieur contenant de 5 à 8 atomes de carbone,

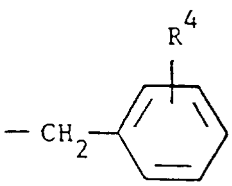 , 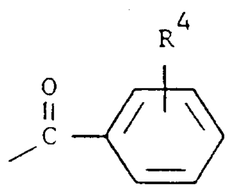 , 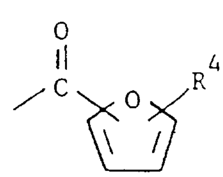 ;

$R^3$ est

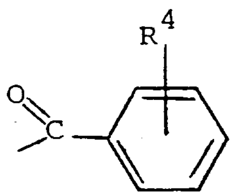

ou

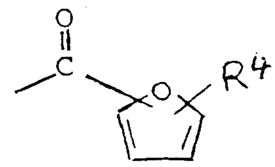

ou $R^2$ et $R^3$ sont liés ensemble pour former $-(CH_2-)_mCO-$, où m est 3 à 6;
ou $R^2$ et $R^3$ sont liés ensemble pour former

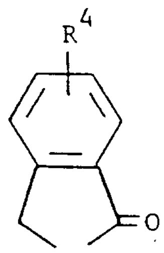

ou

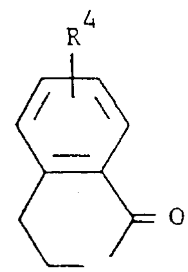

où $R^4$ est choisi parmi le groupe consistant en hydrogène, alcoxy contenant de 1 à 5 atomes de carbone, amino ou amino mono- ou disubstitué par alcoyle, ces groupes alcoyle contenant de 1 à 5 atomes de carbone, et des sels et dérivés solvatés pharmaceutiquement acceptables de celui-ci, qui comprend

a) la réaction d'un composé de formule

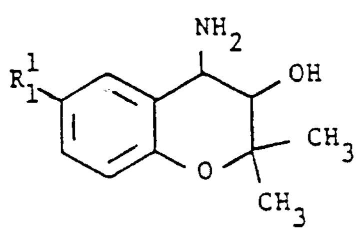

(II)

où $R_1{}^1$ est $R^1$ tel que défini ci-dessus, ou un groupe ou un atome convertible en celui-ci, avec un composé de formule (III)

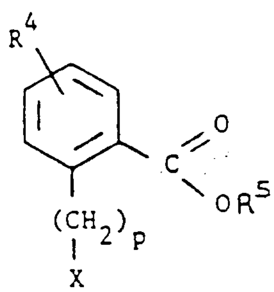

(III)

où X est chlore, brome ou iode; $R^4$ est tel que défini ci-dessus; p est 1 ou 2; $-COOR^5$ est une fonction ester;

et, si nécessaire, la conversion du groupe ou de l'atome $R_1{}^1$ en un groupe tel que défini par $R^1$ ci-dessus, pour donner un composé de formule (I) où $R^2$ et $R^3$ sont liés ensemble pour former

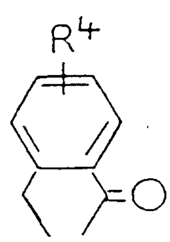

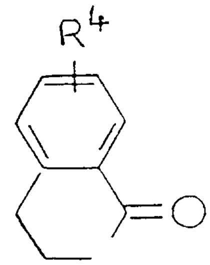

ou

b) la réaction d'un composé de formule

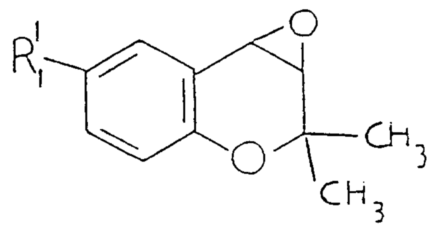

(IV)

où $R_1{}^1$ est tel que défini ci-dessus, avec un composé de formule

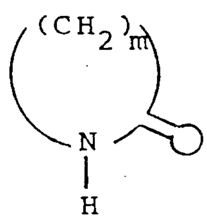

(VIII)

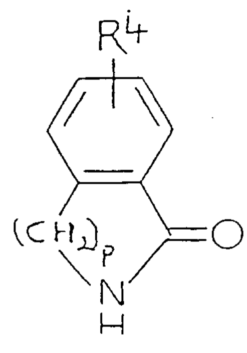

(IX)

ou

$NH_2-(CH_2)_m-COOR^5$ (X)

formules dans lesquelles p est 1 ou 2, m est un entier de 3 à 6, et $-COOR^5$ est une fonction ester,

et, si nécessaire, la conversion du groupe ou de l'atome $R_1{}^1$ en un groupe tel que défini par $R^1$ ci-dessus, pour donner un composé de formule (I) où $R^2$ et $R^3$ sont joints pour former

$-(CH_2)_m-CO-$

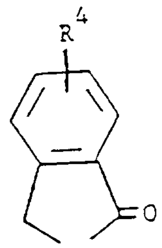

ou

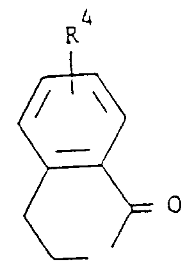

ou

c) l'acylation d'un composé de formule

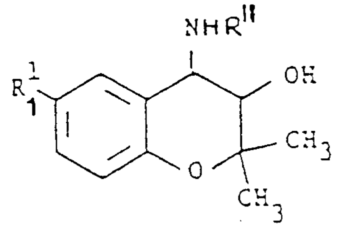

(XI)

où $R_1^1$ est tel que défini ci-dessus et $R^{11}$ est hydrogène, alcoyle en $C_1$-$C_5$, cycloalcoyle en $C_5$-$C_8$,

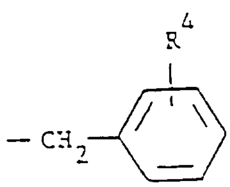

,

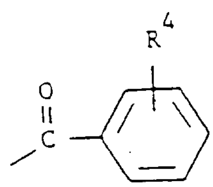

ou

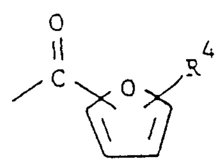

;

avec un agent acylant contenant le groupe $R^{12}$, où $R^{12}$ est

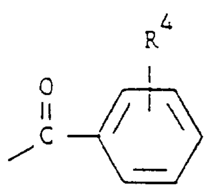

ou

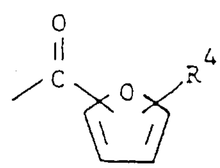

formules dans lesquelles $R^4$ est tel que défini ci-dessus,

et, si nécessaire, la conversion du groupe ou de l'atome $R_1^1$ en un groupe tel que défini par $R^1$ pour donner un composé de formule (I) où $R^2$ est $R^{11}$ tel que défini ci-dessus et $R^3$ est $R^{12}$ tel que défini ci-dessus;

ou

d) la réaction d'un composé de formule (II) tel que défini ci-dessus sous des conditions d'alcoylation réductrices avec un composé de formule

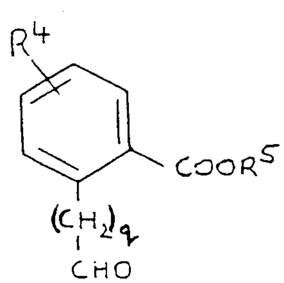

(XII)

où q est 0 ou 1, $R^4$ et $R^5$ sont tels que définis ci-dessus,

et, si nécessaire, la conversion du groupe $R_1{}^1$ en $R^1$ pour donner un composé de formule (I) où $R^2$ et $R^3$ sont liés ensemble pour former

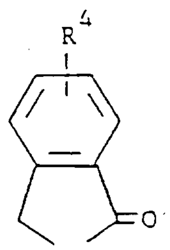

ou

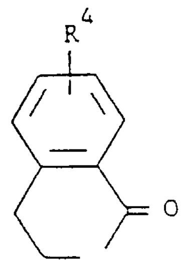

ou

e) la réaction d'un composé de formule

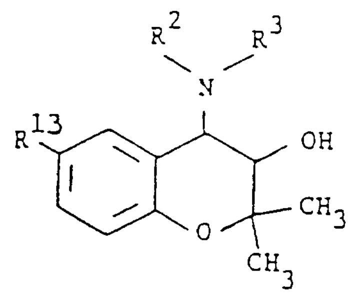

(XIII)

où $R^2$ et $R^3$ sont tels que définis ci-dessus, et $R^{13}$ est un groupe ou un atome qui peut être converti directement en $R^1$, avec un composé qui convertit $R^{13}$ en $R^1$,

ou

f) la conversion d'un composé basique de formule (I) en un sel pharmaceutiquement acceptable de celui-ci par traitement avec un acide ou la neutralisation d'un sel d'addition d'acide avec une base pour donner un composé de formule (I) sous forme de base libre.

**2.** Procédé selon la revendication 1, dans lequel $R^1$ est trifluorométhoxy.

**3.** Procédé selon la revendication 1 ou 2, dans lequel $R^2$ est hydrogène et $R^3$ est

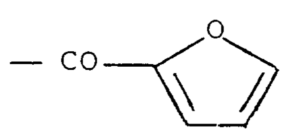

ou $R^2$ et $R^3$ représentent ensemble

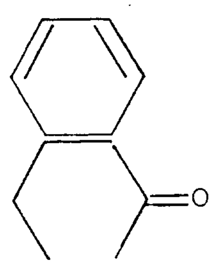

4. Procédé selon la revendication 1, dans lequel le composé préparé est la trans-2-[2,3-dihydro-2,2-diméthyl-3-hydroxy-6-(trifluorométhoxy)-4H-1-benzopyran-4-yl]-2,3-dihydro-1H-isoindol-1-one et les sels pharmaceutiquement acceptables de celle-ci.

5. Procédé selon la revendication 1, dans lequel le composé préparé est le trans-N-[2,3-dihydro-2,2-diméthyl-3-hydroxy-6-(trifluorométhoxy)-4H-1-benzopyran-4-yl]-2-furancarboxamide et les sels pharmaceutiquement acceptables de celui-ci.

6. Procédé selon la revendication 1, dans lequel le composé préparé est la (-)-3S,4R-trans-2-[2,3-dihydro-2,2-diméthyl-3-hydroxy-6-(trifluorométhoxy)-4H-1-benzopyran-4-yl]-2,3-dihydro-1H-isoindol-1-one et les sels pharmaceutiquement acceptables de celle-ci.

7. Procédé pour la résolution d'un racémique de formule (I) tel que défini dans la revendication 1, qui comprend :
   a) la réaction de ce racémique de formule (I) avec un auxiliaire chiral optiquement pur, pour former un mélange de deux diastéréoisomères;
   b) la séparation de ces diastéréoisomères par des moyens physiques, et
   c) l'élimination de l'auxiliaire chiral de l'un des diastéréoisomères ou des deux pour obtenir l'un des énantiomères ou les deux.

8. Procédé suivant la revendication 7, dans lequel l'auxiliaire chiral est de l'isocyanate de 1-(1-naphtyl)-éthyle ou l'isocyanate d'$\alpha$-méthylbenzyle.

9. Procédé pour la préparation d'une composition pharmaceutique comprenant la mise en association d'un composé de formule (I) tel que défini dans la revendication 1 ou d'un sel ou dérivé solvaté pharmaceutiquement acceptable de celui-ci avec un excipient pharmaceutiquement acceptable.

10. Composé de formule (II) tel que représenté dans la revendication 1, procédé a), où $R^1$ est $CF_3O$- ou $CF_3CH_2O$-.

11. Composé de formule (IV) tel que représenté dans la revendication 1, procédé b), où $R^1$ est $CF_3O$- ou $CF_3CH_2O$-.